(19) Europäisches Patentamt
European Patent Office
Office européen des brevets

(11) **EP 1 563 002 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**13.12.2017 Patentblatt 2017/50**

(45) Hinweis auf die Patenterteilung:
**16.07.2014 Patentblatt 2014/29**

(21) Anmeldenummer: **03809325.8**

(22) Anmeldetag: **24.10.2003**

(51) Int Cl.:
*C08J 7/12* (2006.01)  *C08K 3/34* (2006.01)
*A61L 15/00* (2006.01)  *C08F 20/00* (2006.01)
*C08F 8/00* (2006.01)  *B29B 7/00* (2006.01)
*B01F 3/12* (2006.01)  *B01F 13/10* (2006.01)
*B01J 19/18* (2006.01)  *A61L 15/60* (2006.01)
*B01F 7/00* (2006.01)  *B01F 9/00* (2006.01)

(86) Internationale Anmeldenummer:
**PCT/EP2003/011828**

(87) Internationale Veröffentlichungsnummer:
**WO 2004/037903 (06.05.2004 Gazette 2004/19)**

(54) **ABSORBIERENDE POLYMERGEBILDE MIT VERBESSERTER RETENTIONSKAPAZITÄT UND PERMEABILITÄT**

ABSORBENT POLYMER STRUCTURE PROVIDED WITH AN IMPROVED RETENTION CAPACITY AND PERMEABILITY

MATIERE POLYMERE ABSORBANTE A CAPACITE DE RETENTION ET PERMEABILITE AMELIOREES

(84) Benannte Vertragsstaaten:
**AT BE BG CH CY CZ DE DK EE ES FI FR GB GR HU IE IT LI LU MC NL PT RO SE SI SK TR**

(30) Priorität: **25.10.2002 DE 10249821**
**25.10.2002 DE 10249822**

(43) Veröffentlichungstag der Anmeldung:
**17.08.2005 Patentblatt 2005/33**

(73) Patentinhaber: **Evonik Degussa GmbH**
**45128 Essen (DE)**

(72) Erfinder:
• **HARREN, Jörg**
**47807 Krefeld (DE)**
• **BREHM, Helmut**
**47800 Krefeld (DE)**
• **KERKMANN, Andreas**
**47574 Goch (DE)**
• **RAMLOW, Stephan**
**47807 Krefeld (DE)**

(56) Entgegenhaltungen:
EP-A- 1 211 266    EP-A1- 0 480 031
EP-A1- 0 775 593    EP-A1- 1 325 777
EP-A1- 1 325 777    EP-A2- 0 450 924
EP-A2- 1 063 002    WO- -03//002623

WO-A-01/13841    WO-A1-00/22018
WO-A1-00/53664    WO-A1-01/66056
WO-A1-01/74913    WO-A1-01/90229
WO-A1-02/20068    WO-A1-02/20678
WO-A1-02/22717    WO-A1-92/00108
WO-A1-95/11932    WO-A1-95/22356
WO-A1-95/26209    WO-A1-98/48857
WO-A1-2006/033477    DE-A- 3 503 458
DE-A1- 4 015 085    JP-A- H0 616 822
JP-A- H04 120 176    JP-A- H11 349 625
JP-A- 2001 137 704    US-A- 5 140 076
US-A- 5 156 707    US-A- 5 284 900
US-A- 5 416 174    US-A1- 2002 128 396
US-A1- 2002 128 618    US-A1- 2002 165 288
US-B1- 6 228 816

• Annonymous: "Centrifuge Retention capacity 441.1-99 (Recommended Test Method: Polyacrylate Superabsorbent Powders; Centrifuge Retention Capacity in Saline by Gravimetric determination)", , 1 February 1999 (1999-02-01), pages 1-5, XP055071837, [retrieved on 2013-07-18]
• Annonymous: "Absorbancy Against pressure 442.1-99 (Recommended Test method; Superabsorbent Materials; Polyacrylate Superabsorbent Powders; Absorbency Against Pressure by Gravimetric Determination)", , 1 February 1999 (1999-02-01), pages 1-5, XP055071838, [retrieved on 2013-07-18]

• Annonymous: "Kieselsol", RÖMPP Online, Version 3.34, 1 December 2006 (2006-12-01), page 1, XP055072476, Retrieved from the Internet: URL:http://www.roempp.com/ [retrieved on 2013-07-23]

**Beschreibung**

Die Erfindung betrifft ein Verfahren zur Herstellung eines absorbierenden Polymergebildes.

Superabsorber sind wasserunlösliche, vernetzte Polymere, die in der Lage sind, unter Quellung und Ausbildung von Hydrogelen große Mengen an wässrigen Flüssigkeiten, insbesondere Körperflüssigkeiten, vorzugsweise Urin oder Blut, aufzunehmen und unter einem bestimmten Druck zurückzuhalten. Durch diese charakteristischen Eigenschaften finden diese Polymere hauptsächlich Anwendung bei der Einarbeitung in Sanitärartikeln, wie beispielsweise Babywindeln, Inkontinenzprodukten oder Damenbinden.

Bei den gegenwärtig kommerziell verfügbaren Superabsorbern handelt es sich im wesentlichen um vernetzte Polyacrylsäuren oder vernetzte Stärke-Acrylsäure-Pfropfpolymerisate, bei denen die Carboxylgruppen teilweise mit Natronlauge oder Kalilauge neutralisiert sind.

Aus ästhetischen Gründen und aus Umweltaspekten besteht zunehmend die Tendenz, die Sanitärartikel immer kleiner und dünner zu gestalten. Um ein gleichbleibendes Gesamtretentionsvermögen der Sanitärartikel zu gewährleisten, kann dieser Anforderung nur durch Reduktion des Anteils an großvolumigen Fluff entsprochen werden. Hierdurch fallen dem Superabsorber weitere Aufgaben hinsichtlich Transport und Verteilung von Flüssigkeit zu, die sich als Permeabilitätseigenschaften zusammenfassen lassen.

Unter Permeabilität versteht man bei Superabsorbermaterialien die Fähigkeit, im gequollenen Zustand zugegebene Flüssigkeiten zu transportieren und dreidimensional zu verteilen. Dieser Prozess läuft im gequollenen Superabsorbergel über kapillaren Transport durch Zwischenräume zwischen den Gelpartikeln ab. Ein Flüssigkeitstransport durch gequollene Superabsorberpartikel selbst folgt den Gesetzen der Diffusion und ist ein sehr langsamer Prozess, der in der Nutzungssituation des Sanitärartikels keine Rolle bei der Verteilung der Flüssigkeit spielt. Bei Superabsorbermaterialien, die einen kapillaren Transport aufgrund mangelnder Gelstabilität nicht bewerkstelligen können, wurde durch Einbetten dieser Materialien in eine Fasermatrix eine Separation der Partikel voneinander unter Vermeidung des Gel-Blocking-Phänomens sichergestellt. In Windelkonstruktionen neuer Generation befindet sich in der Absorberschicht nur wenig oder überhaupt kein Fasermaterial zur Unterstützung des Flüssigkeitstransports. Die hier verwendeten Superabsorber müssen demnach eine ausreichend hohe Stabilität im gequollenen Zustand besitzen, damit das gequollene Gel noch eine ausreichende Menge an kapillaren Räumen besitzt, durch die Flüssigkeit transportiert werden kann.

Um Superabsorbermaterialien mit hoher Gelstabilität zu erhalten, kann einerseits der Grad der Vernetzung des Polymers angehoben werden, was zwangsläufig eine Verminderung der Quellfähigkeit und des Retentionsvermögens zur Folge hat. Eine optimierte Kombination von verschiedenen Vernetzern und Comonomeren, wie in DE 196 46 484 beschrieben, vermag die Permeabilitätseigenschaften zwar verbessern, nicht aber auf ein Niveau, das beispielsweise den Einbau einer gegebenenfalls nur aus Superabsorbern bestehende Schicht in eine Windelkonstruktion erlaubt.

Weiterhin können Methoden zur Nachbehandlung der Oberfläche von Polymerpartikeln zur Verbesserung der Superabsorbereigenschaften zum Einsatz kommen. Als Oberflächenbehandlung sind beispielsweise Nachvernetzung des absorbierenden Polymergebildes an der Oberfläche, das in Kontakt bringen der Oberfläche mit anorganischen Verbindungen oder aber die Nachvernetzung der Oberfläche in Gegenwart anorganischer Verbindungen aus dem Stand der Technik bekannt.

So beschreiben EP-A-0 450 923, EP-A-0 450 922, DE-A-35 23 617, US 5,140,076 und US 4,734,478 die Behandlung der Oberfläche absorbierender Polymere durch in Kontakt bringen der Oberfläche mit anorganischen Verbindungen, wie beispielsweise mit feinverteilter Kieselerde, während oder nach der Nachvernetzung der Oberfläche. Neben einer erhöhten Absorptionsgeschwindigkeit unter Druck wird auch eine erhöhte Permeabilität der absorbierenden Polymere durch diese Art der Oberflächenbehandlung erzielt.

Die DE 35 03 458 beschreibt ein Verfahren zur Herstellung eines verbesserten absorbierenden Harzes, bei dem ein wasserabsorbierendes Harz, das Einheiten eines Monomers mit einer Carboxylgruppe in Form der freien Säure oder eines Salzes als eine Aufbaukomponente desselben enthält, in Gegenwart eines Pulvers aus einem feinteiligen Metalloxid ein Vernetzungsmittel und Wasser absorbieren lässt und die resultierende Mischung unter Rühren erhitzt, um die Vernetzung des Harzes und die Entfernung von Wasser zu bewirken. Hier werden absorbierende Harze mit einem guten Wasserabsoptionsvermögen erhalten, die zugleich eine gute Absorptionsgeschwindigkeit aufweisen.

US 4,535,098 beschreibt ein Verfahren zur Erhöhung der Gelstärke von nicht nachvernetzten Superabsorbern durch Quellung von absorbierenden Polymeren in Gegenwart einer kolloiddispersen, anorganischen Verbindung, wie etwa einem Kieselsäuresol, oder aber durch Herstellung eines absorbierenden Polymers in Gegenwart einer kolloiddispersen, anorganischen Verbindung.

DE 198 05 447 offenbart ein Verfahren zur Nachvernetzung von Polyacrylnitrilhydrolysaten mit bifunktionellen Verbindungen und eine gleichzeitige Immobilisierung von Kieselsäure in der Oberflächenstruktur der superabsorbierenden Polymers. Die Kieselsäure wurde dabei zusammen mit dem Vernetzungsmittel in einem Wasser/Alkohol-Gemisch in Kontakt mit der Oberfläche gebracht. Durch die Immobilisierung der Kieselsäure soll eine Verbesserung der *Absorbency under Load* sowie eine Verringerung des Gelblockings erreicht werden.

DE 198 54 575 beschreibt den Zusatz von Alkalisalzen der Kieselsäure vor, während oder nach der Polymerisation oder zur teilweisen Neutralisation des Superabsorbers. Durch diese Oberflächenbehandlung wird eine verbesserte Permeabilität erreicht, die jedoch hauptsächlich auf die durch nichtquellbaren Zusatz bedingte verringerte Retention der Polymere zurückzuführen ist.

US 5,147,921 offenbart den Zusatz eines Kieselsäuresols als inertes Füllmittel, dass sich in der zu polymerisierenden Monomerlösung dispergieren lässt.

EP 1211266 A1 beschreibt ein Verfahren zur Herstellung von superabsorbierenden Polymerisaten, bei dem man vernetzte und/oder unvernetzte wässrige Polyacrylnitril-Emulsionen auf Basis von Homo- und/oder Copolymerisaten durch Reaktion mit einer Alkalihydroxidlösung in hochkonzentrierten Reaktionsgemischen adiabatisch ohne Zuführung von mechanischer oder thermischer Energie bei einer Anfangstemperatur von 10 °C bis 40 °C zur Reaktion bringt. In EP 1211266 A1 wird außerdem noch auf eine optionale Oberflächenmodifizierung hingewiesen, um die Anti-Gelblocking-Eigenschaften zu verbessern. Die betreffende Modifizierung sieht vor, dass die gemahlenen und klassierten Teilchen in einem Wasser/Alkoholgemisch mit einem aldehydischen Vernetzungsmittel und in Gegenwart von Kieselsäure behandelt werden. Dabei wird ein überaus großer Überschuss Methanol eingesetzt. Polymere auf Polyacrylsäurebasis werden in EP 1211266 dagegen nicht beschrieben und ebenso wenig wird der Gebrauch von Kieselsäuresol beschrieben.

WO 01/13841 A1 beschreibt ein absorptionsfähiges, vernetztes Polymerisat für Wasser oder wässrige Körperflüssigkeiten, auf Basis von gegebenenfalls teilneutralisierten, monoethylenisch ungesättigten Säuregruppen tragenden Monomeren, wobei das Polymerisat Cyclodextrin oder Cyclodextrinderivate und siliziumreiche Zeolithe mindestens teilweise kovalent, ionisch gebunden oder darin eingeschlossen enthält. Als siliziumreicher Zeolith wird z.B. Flavith S 108 eingesetzt. Der Einsatz von Kieselsäuresol ist dagegen nicht vorgesehen.

JP 1994-16822 beschreibt die Nachbehandlung der Oberfläche von absorbierenden Polymeren mit einem anorganischen SoL Um eine verbesserte Verarbeitbarkeit des zur Bildung von Agglomeraten neigenden Gemisches zu ermöglichen, wird zusätzlich eine organische Lösungsmittelkomponente zugegeben. Als organische Lösungsmittelkomponente werden beispielsweise Mono- und Dimethylether von Diolen oder Diole selbst genannt. Nach der Trocknung sollen die absorbierenden Polymere eine höhere Gelstabilität, eine geringere Neigung zum Gelblocking und eine verbesserte Permeabilität für Wasser in einfachen Tests ohne Druckbelastung der Superabsorber aufweisen.

Der Stand der Technik beschreibt Verfahren, bei dem anorganische Partikel entweder trocken mit dem Superabsorber abgemischt werden oder mit Hilfe großer zum Teil organischer Lösungsmittelmengen in den Prozess der Nachvernetzung eingebracht werden, um eine Agglomeration der Superabsorberteilchen zu verhindern. Diese Verfahren weisen jedoch den Nachteil auf, dass entweder große Lösungsmittelmengen gehandhabt werden müssen, was sowohl aus ökonomischen als auch aus ökologischen Gründen unerwünscht ist. Zudem neigen superabsorbierende Polymere beim Mischen mit großen Flüssigkeitsmengen zur Agglomeration, was die Verarbeitbarkeit innerhalb eines kontinuierlichen Herstellungsprozesses stark beeinträchtigen kann. Eine einfache Abmischung mit anorganischen, feinteiligen Substanzen bringt hingegen Nachteile, wie etwa Entmischung oder Stauben, mit sich. Die Zugabe von anorganischen Additiven in wässrigen Lösungen zur Nachvernetzung selbst ist schwierig, da sich die anorganischen Partikel schnell wieder absetzen. Zudem lassen sich anorganische Dispersionen schlecht dosieren.

Bedingt durch die Anwesenheit der im Stand der Technik offenbarten feinteiligen, anorganischen Substanzen kommt es zu einer inhomogenen Verteilung des chemischen Nachvernetzers auf der Oberfläche der absorbierenden Polymere und demnach auch zu einer inhomogenen Nachvernetzung. Dies wiederum führt dazu, dass superabsorbierende Polymere mit einer unbefriedigenden Gesamtperformance vor allem hinsichtlich der Retention und der Permeabilität erhalten werden. Eine homogene Verteilung ist bei den im Stand der Technik beschriebenen Verfahren zur Oberflächenbehandlung allenfalls durch die Verwendung große Mengen einer wässrigen oder alkoholischen Lösung enthaltend den chemischen Vernetzer möglich.

Allgemein liegt der Erfindung die Aufgabe zugrunde, die sich aus dem Stand der Technik ergebenden Nachteile zu überwinden.

Ferner besteht eine erfindungsgemäße Aufgabe darin, superabsorbierende Polymere zur Verfugung zu stellen, die als Eigenschaftskombination nicht nur eine hohe Aufnahmekapazität unter Druck, sondern auch die üblicherweise gegenläufigen Eigenschaften eines hohen Retentionsvermögens und einer guten Permeabilität in sich vereinigen, um den Anforderungen moderner Hygieneartikeln, insbesondere Windeln, Inkontinentsprodukten oder Damenbinden, an absorbierende Polymere gerecht zu werden. Insbesondere sollen diese Polymere möglichst geringe Mengen an toxischen Monomeren, wie etwa Acrylamid oder Acrylnitril, beinhalten, die bei einem Kontakt der superabsorbierenden Polymere mit Körperflüssigkeiten ausgewaschen und, beispielsweise im Falle eines Einsatzes der superabsorbierenden Polymere in Windeln, auf diese Weise in Kontakt mit der Haut des Windelträgers treten können.

Eine weitere, der vorliegenden Erfindung zugrunde liegende Aufgabe bestand darin, Hygieneartikel wie beispielsweise Windeln bereitzustellen, die im Vergleich zu den aus dem Stand der Technik bekannten Hygieneartikeln besser in der Lage sind, aufgenommene Körperflüssigkeiten zurückzuhalten, unter Druck Flüssigkeiten aufzunehmen und bei der Aufnahme von Flüssigkeiten diese möglichst schnell und gleichmäßig im Hygieneartikel zu verteilen.

Zudem liegt eine andere erfindungsgemäße Aufgabe darin, ein Verfahren zu schaffen, mit dem solche absorbierenden Polymere in einfacher, kontinuierlicher Weise möglichst geringen Mengen organischer Lösungsmittel darstellbar sind. Bei diesem Herstellungsverfahren sollten sich zugesetzte anorganische Hilfsstoffe maximal in geringen Mengen vom superabsorbierenden Polymer ablösen, die die Polymereigenschaften nicht nachteilig beeinflussen. Die in diesem Verfahren zur Behandlung der Oberfläche des absorbierenden Polymers eingesetzte Lösung sollte wie ein Einphasensystem gehandhabt werden können und sich gleichmäßig dosieren lassen. Der beschichtete Superabsorber sollte im Verlaufe des Verfahrens in nur geringfügigen Umfang Agglomerate bilden und sollte in einfacher Weise einem kontinuierlich arbeitenden Temperungsschritt zugeführt werden können.

Die vorstehenden Aufgaben werden gelöst durch ein Verfahren nach Anspruch 1.

Die vorstehenden Aufgaben werden auch gelöst durch ein Verfahren nach Anspruch 2.

Hier beschriebene absorbierende Polymergebilde (Pa) sind Fasern, Schäume oder Teilchen, wobei Fasern und Teilchen bevorzugt und Teilchen besonders bevorzugt sind. Absorbierende Polymergebilde (Pa) in diesen Formen werden erhalten, in dem als absorbierende Polymergebilde (Pu1) oder (Pu2) in entsprechender Weise Fasern, Schäume oder Teilchen eingesetzt werden.

Erfindungsgemäss bevorzugte absorbierende Polymerfasern sind so dimensioniert, dass sie in oder als Garne für Textilien und auch direkt in Textilien eingearbeitet werden können. Es ist erfindungsgemäss bevorzugt, dass die absorbierenden Polymerfasern eine Länge im Bereich von 1 bis 500, bevorzugt 2 bis 500 und besonders bevorzugt 5 bis 100 mm und einen Durchmesser im Bereich von 1 bis 200, bevorzugt 3 bis 100 und besonders bevorzugt 5 bis 60 Denier besitzen.

Erfindungsgemäss besonders bevorzugte absorbierende Polymerteilchen sind so dimensioniert, dass sie eine mittlere Teilchengröße gemäss ERT 420.1-99 im Bereich von 10 bis 3000, vorzugsweise 20 bis 2000 und besonders bevorzugt 150 bis 850 $\mu$m aufweisen.

Das im erfindungsgemäßen Verfahren eingesetzte absorbierende Polymergebilde (Pu1) bzw. (Pu2) ist bevorzugt ein Polymergebilde, welches auf

($\alpha$1) 55 bis 98,99 Gew.-% und besonders bevorzugt 70 bis 98,79 Gew.-% polymerisierten, ethylenisch ungesättigten, säuregruppenhaltigen Monomeren oder deren Salze oder polymerisierten, ethylenisch ungesättigten, einen protonierten oder quarternierten Stickstoff beinhaltenden Monomeren, oder deren Mischungen, wobei mindestens ethylenisch ungesättigte, säuregruppenhaltige Monomere, vorzugsweise Acrylsäure, beinhaltende Mischungen besonders bevorzugt sind,

($\alpha$2) 0-80 Gew.-%, vorzugsweise 0-44,99 Gew.-% und besonders bevorzugt 0,1-44,89 Gew.-% polymerisierten, monoethylenisch ungesättigten, mit ($\alpha$1) copolymerisierbaren Monomeren,

($\alpha$3) 0,001-5 Gew.-%, vorzugsweise 0,01-3 Gew.-% und besonders bevorzugt 0,01-2,5 Gew.-% eines oder mehrerer Vernetzer,

($\alpha$4) 0-30 Gew.-%, vorzugsweise 0-5 Gew.-% und besonders bevorzugt 0,1-5 Gew.-% eines wasserlöslichen Polymeren, sowie

($\alpha$5) 0-20 Gew.-%, vorzugsweise 0 bis 10 Gew.-% und besonders bevorzugt 0,1-8 Gew.-% eines oder mehrerer Hilfsmittel basiert, wobei die Summe der Gewichtsmengen ($\alpha$1) bis ($\alpha$5) 100 Gew.-% beträgt.

Die monoethylenisch ungesättigten, säuregruppenhaltigen Monomere ($\alpha$1) sind teilweise neutralisiert. Vorzugsweise sind die monoethylenisch ungesättigten, säuregruppenhaltigen Monomere zu mindestens 25 Mol%, besonders bevorzugt zu mindestens 50 Mol% und darüber hinaus bevorzugt zu 50-80 Mol% neutralisiert. In diesem Zusammenhang wird auf DE 195 29 348 verwiesen. Die Neutralisation kann teilweise oder ganz auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak und Alkalimetallhydroxiden, besonders bevorzugt mit Natriumhydroxid und mit Ammoniak.

Ferner können bei einem Polymer die freien Säuregruppen überwiegen, so dass dieses Polymer einen im sauren Bereich liegenden pH-Wert aufweist. Dieses saure wasserabsorbierende Polymer kann durch ein Polymer mit freien basischen Gruppen, vorzugsweise Amingruppen, das im Vergleich zu dem sauren Polymer basisch ist, mindestens teilweise neutralisiert werden. Diese Polymere werden in der Literatur als *"Mixed-Bed Ion-Exchange Absorbent Polymers"* (MBIEA-Polymere) bezeichnet und sind unter anderem in der WO 99/34843 offenbart. Auf die Offenbarung der WO 99/34843 wird hiermit verwiesen. In der Regel stellen MBIEA-Polymere eine Zusammensetzung dar, die zum einen basische Polymere, die in der Lage sind, Anionen auszutauschen, und andererseits ein im Vergleich zu dem basischen Polymer saures Polymer, das in der Lage ist, Kationen auszutauschen, beinhalten. Das basische Polymer weist basische Gruppen auf und wird typischerweise durch die Polymerisation von Monomeren erhalten, die basische Gruppen oder Gruppen tragen, die in basische Gruppen umgewandelt werden können. Bei diesen Monomeren handelt es sich vor allen Dingen um solche, die primäre, sekundäre oder tertiäre Amine oder die entsprechenden Phosphine oder mindestens zwei der vorstehenden funktionellen Gruppen aufweisen. Zu dieser Gruppe von Monomeren gehören insbesondere Ethylenamin, Allylamin, Diallylamin, 4-Aminobuten, Alkyloxycycline, Vinylformamid, 5-Aminopenten, Carbodiimid, Formaldacin, Melamin und dergleichen, sowie deren sekundäre oder tertiäre Aminderivate.

Die monoethylenisch ungesättigten, säuregruppenhaltigen Monomere ($\alpha$1) sind teilweise neutralisiert. Vorzugsweise sind die monoethylenisch ungesättigten, säuregruppenhaltigen Monomere zu mindestens 25 Mol%, besonders bevorzugt zu mindestens 50 Mol% und darüber hinaus bevorzugt zu 50-90 Mol% neutralisiert. Die Neutralisation der Monomere ($\alpha$1) kann vor auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak oder mit Alkalimetallhydroxiden, besonders bevorzugt mit Natriumhydroxid oder mit Ammoniak.

Bevorzugte monoethylenisch ungesättigte, säuregruppenhaltige Monomere ($\alpha$1) sind Acrylsäure, Methacrylsäure, Ethacrylsäure, $\alpha$-Chloracrylsäure, $\alpha$-Cyanoacrylsäure, $\beta$-Methylacrylsäure (Crotonsäure), $\alpha$-Phenylacrylsäure, $\beta$-Acryloxypropionsäure, Sorbinsäure, $\alpha$-Chlorsorbinsäure, 2'-Methylisocrotonsäure, Zimtsäure, p-Chlorzimtsäure, $\beta$-Stearylsäure, Itaconsäure, Citraconsäure, Mesaconsäure, Glutaconsäure, Aconitsäure, Maleinsäure, Fumarsäure, Tricarboxyethylen und Maleinsäureanhydrid, wobei Acrylsäure sowie Methacrylsäure besonders und Acrylsäure darüber hinaus bevorzugt sind.

Neben diesen carboxylatgruppenhaltigen Monomeren sind als monoethylenisch ungesättigte, säuregruppenhaltige Monomere ($\alpha$1) des Weiteren ethylenisch ungesättigte Sulfonsäuremonomere oder ethylenisch ungesättigte Phosphonsäuremonomere bevorzugt.

Ethylenisch ungesättigte Sulfonsäuremonomere sind Allylsulfonsäure oder aliphatische oder aromatische Vinylsulfonsäuren oder acrylische oder methacrylische Sulfonsäuren bevorzugt. Als aliphatische oder aromatische Vinylsulfonsäuren sind Vinylsulfonsäure, 4-Vinylbenzylsulfonsäure, Vinyltoluolsulfonsäure und Styrolsulfonsäure bevorzugt. Als Acryl- bzw. Methacrylsulfonsäuren sind Sulfoethyl(meth)acrylat, Sulfopropyl(meth)acrylat, 2-Hydroxy-3-rnethacryloxypropylsulfonsäure und 2-Acrylamido-2-methylpropansulfonsäure bevorzugt.

Ferner sind ethylenisch ungesättigte Phosphonsäuremonomere, wie Vinylphosphonsäure, Allylphosphonsäure, Vinylbenzylphosponsäure, (Meth)acrylamidoalkylphosphonsäuren, Acrylamidoalkyldiphosphonsäuren, phosphonomethylierte Vinylamine und (Meth)acrylphosphonsäurederivate bevorzugt.

Als ethylenisch ungesättigte, einen protonierten Stickstoff enthaltende Monomere ($\alpha$1) sind vorzugsweise Dialkylaminoalkyl(meth)acrylate in protonierter Form, beispielsweise Dimethylaminoethyl(meth)acrylat-Hydrochlorid oder Dimethylaminoethyl(meth)acrylat-Hydrosulfat, sowie Dialkylaminoalkyl(meth)acrylamide in protonierter Form, beispielsweise Dimethylaminoethyl(meth)acrylamid-Hydrochlorid oder Dimethylaminoethyl(meth)acrylamid-Hydrosulfat bevorzugt.

Als ethylenisch ungesättigte, einen quarternierten Stickstoff enthaltende Monomere ($\alpha$1) sind Dialkylammoniumalkyl(meth)acrylate in quarternisierter Form, beispielsweise Trimethylammoniumethyl(meth)acrylat-Methosulfat oder Dimethylethylammoniumethyl(meth)acrylat-Ethosulfat sowie (Meth)acrylamidoalkyldialkylamine in quarternisierter Form, beispielsweise (Meth)acrylamidopropyltrimethylammoniumchlorid und (Meth)acrylamidopropyltrimethylammoniumsulfat bevorzugt.

Erfindungsgemäß besteht die Komponente ($\alpha$1) zu mindestens 50 Gew.-%, vorzugsweise zu mindestens 70 Gew.-% und darüber hinaus bevorzugt zu mindestens 90 Gew.-% aus carboxylatgruppenhaltigen Monomeren. Erfindungsgemäß besteht die Komponente ($\alpha$1) zu mindestens 50 Gew.-%, vorzugsweise zu mindestens 70 Gew.-% aus Acrylsäure, die zu mindestens 20 Mol-%, besonders bevorzugt zu mindestens 50 Mol-% neutralisiert ist.

Als monoethylenisch ungesättigte, mit ($\alpha$1) copolymerisierbare Monomere ($\alpha$2) sind Acrylamide und Methacrylamide bevorzugt.

Mögliche (Meth)acrylamide sind neben Acrylamid und Methacrylamid alkylsubstituierte (Meth)acrylamideoder aminoalkylsubstituierte Derivate des (Meth)acrylamids, wie N-Methylol(meth)acrylamid, N,N-Dimethylamino(meth)acrylamid, Dimethyl(meth)acrylamid oder Diethyl(meth)acrylamid. Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Des Weiteren sind als monoethylenisch ungesättigte, mit ($\alpha$1) copolymerisierbaren Monomere ($\alpha$2) in Wasser dispergierbare Monomere bevorzugt. Als in Wasser dispergierbare Monomere sind Acrylsäureester und Methacrylsäureester, wie Methyl(meth)acrylat, Ethyl(meth)acrylat, Propyl(meth)acrylat oder Butyl(meth)acrylat, sowie Methylpolyethylenglykol(meth)acrylat, Methylpolyethylenglykolallylether, Vinylacetat, Styrol und Isobutylen bevorzugt.

Vernetzer ($\alpha$3) sind Verbindungen, die mindestens zwei ethylenisch ungesättigte Gruppen innerhalb eines Moleküls aufweisen (Vernetzerklasse I), Verbindungen, die mindestens zwei funktionelle Gruppen aufweisen, die mit funktionellen Gruppen der Monomeren ($\alpha$1) oder ($\alpha$2) in einer Kondensationsreaktion (=Kondensationsvernetzer), in einer Additionsreaktion oder in einer Ringöffnungsreaktion reagieren können (Vernetzerklasse II), Verbindungen, die mindestens eine ethylenisch ungesättigte Gruppe und mindestens eine funktionelle Gruppe, die mit funktionellen Gruppen der Monomeren ($\alpha$1) oder ($\alpha$2) in einer Kondensationsreaktion, in einer Additionsreaktion oder in einer Ringöffnungsreaktion reagieren kann (Vernetzerklasse III), aufweisen, oder polyvalente Metallkationen (Vernetzerklasse IV).

Dabei wird durch die Verbindungen der Vernetzerklasse I eine Vernetzung der Polymere durch die radikalische Polymerisation der ethylenisch ungesättigten Gruppen des Vernetzermoleküls mit den monoethylenisch ungesättigten Monomeren (α1) oder (α2) erreicht, während bei den Verbindungen der Vernetzerklasse II und den polyvalenten Metallkationen der Vernetzerklasse IV eine Vernetzung der Polymere durch Kondensationsreaktion der funktionellen Gruppen (Vernetzerklasse II) bzw. durch elektrostatische Wechselwirkung des polyvalenten Metallkations (Vernetzerklasse IV) mit den funktionellen Gruppen der Monomere (α1) oder (α2) erreicht wird. Bei den Verbindungen der Vernetzerklasse III erfolgt dementsprechend eine Vernetzung des Polymers sowohl durch radikalische Polymerisation der ethylenisch ungesättigten Gruppe als auch durch Kondensationsreaktion zwischen der funktionellen Gruppe des Vernetzers und den funktionellen Gruppen der Monomeren (α1) oder (α2).

Bevorzugte Verbindungen der Vernetzerklasse I sind Poly(meth)acrylsäureester oder Poly(meth)acrylamide, die beispielsweise durch die Umsetzung eines Polyols, wie beispielsweise Ethylenglykol, Propylenglykol, Trimethylolpropan, 1,6-Hexandiol, Glycerin, Pentaerythrit, Polyethylenglykol oder Polypropylenglykol, eines Aminoalkohols, eines Polyalkylenpolyamins, wie beispielsweise Diethylentriamin oder Triethylentetraamin, oder eines alkoxylierten Polyols mit Acrylsäure oder Methacrylsäure gewonnen werden. Als Verbindungen der Vernetzerklasse I sind des Weiteren Polyvinylverbindungen, Poly(meth)allylverbindungen, (Meth)acrylsäureester einer Monovinylverbindung oder (Meth)acrylsäureester einer Mono(meth)allylverbindung, vorzugsweise der Mono(meth)allylverbindungen eines Polyols oder eines Aminoalkohols, bevorzugt. In diesem Zusammenhang wird auf DE 195 43 366 und DE 195 43 368 verwiesen.

Als Verbindungen der Vernetzerklasse I seien als Beispiel genannt Alkenyldi(meth)acrylate, beispielsweise Ethylenglykoldi(meth)acrylat, 1,3-Propylenglykoldi(meth)acrylat, 1,4-Butylenglykoldi(meth)acrylat, 1,3-Butylenglykoldi(meth)acrylat, 1,6-Hexandioldi(meth)acrylat, 1,10-Decandioldi(meth)acrylat, 1,12-Dodecandioldi(meth)acrylat, 1,18-Octadecandioldi(meth)acrylat, Cyclopentandioldi(meth)acrylat, Neopentylglykoldi(meth)acrylat, Methylendi(meth)acrylat oder Pentaerythritdi(meth)acrylat, Alkenyldi(meth)acrylamide, beispielsweise N-Methyldi(meth)acrylamid, N,N'-3-Methylbutylidenbis(meth)acrylamid, N,N'-(1,2-Di-hydroxyethylen)bis(meth)acrylamid, N,N'-Hexamethylenbis(meth)acrylamid oder N,N'-Methylenbis(meth)acrylamid, Polyalkoxydi(meth)acrylate, beispielsweise Diethylenglykoldi(meth)acrylat, Triethylenglykoldi(meth)acrylat, Tetraethylenglykoldi(meth)acrylat, Dipropylenglykoldi(meth)acrylat, Tripropylenglykoldi(meth)acrylat oder Tetrapropylenglykoldi(meth)acrylat, Bisphenol-A-di(meth)acrylat, ethoxyliertes Bisphenol-A-di(meth)acrylat, Benzylidindi(meth)acrylat, 1,3-Di(meth)acryloyloxy-propanol-2, Hydrochinondi(meth)acrylat, Di(meth)acrylatester des vorzugsweise mit 1 bis 30 Mol Alkylenoxid pro Hydroxylgruppe oxyalkylierten, vorzugsweise ethoxylierten, Trimethylolpropans, Thioethylenglykoldi(meth)acrylat, Thiopropylenglykoldi(meth)acrylat, Thiopolyethylenglykoldi(meth)acrylat, Thiopolypropylenglykoldi(meth)acrylat, Divinylether, beispielsweise 1,4-Butandioldivinylether, Divinylester, beispielsweise Divinyladipat, Alkandiene, beispielsweise Butadien oder 1,6-Hexadien, Divinylbenzol, Di(meth)allylverbindungen, beispielsweise Di(meth)allylphthalat oder Di(meth)allylsuccinat, Homo- und Copolymere von Di(meth)allyldimethylammoniumchlorid und Homo- und Copolymere von Diethyl(meth)allylaminomethyl(meth)acrylatammoniumchlorid, Vinyl-(meth)acryl-Verbindungen, beispielsweise Vinyl(meth)acrylat, (Meth)allyl-(meth)acryl-Verbindungen, beispielsweise (Meth)allyl(meth)acrylat, mit 1 bis 30 Mol Ethylenoxid pro Hydroxylgruppe ethoxyliertes (Meth)allyl(meth)acrylat, Di(meth)allylester von Polycarbonsäuren, beispielsweise Di(meth)allylmaleat, Di(meth)allylfumarat, Di(meth)allylsuccinat oder Di(meth)allylterephthalat, Verbindungen mit 3 oder mehr ethylenisch ungesättigten, radikalisch polymerisierbaren Gruppen wie beispielsweise Glycerintri(meth)acrylat, (Meth)acrylatester des mit vorzugsweise 1 bis 30 Mol Ethylenoxid pro Hydroxylgruppe oxyethylierten Glycerins, Trimethylolpropantri(meth)acrylat, Tri(meth)acrylatester des vorzugsweise mit 1 bis 30 Mol Alkylenoxid pro Hydroxylgruppe oxyalkylierten, vorzugsweise ethoxylierten Trimethylolpropans, Trimethacrylamid, (Meth)allylidendi(meth)acrylat, 3-Allyloxy-1,2-propandioldi(meth)acrylat, Tri(meth)allylcyanurat, Tri(meth)allylisocyanurat, Pentaerythrittetra(meth)acrylat, Pentaerythrittri(meth)acrylat, (Meth)acrylsäureester des mit vorzugsweise 1 bis 30 Mol Ethylenoxid pro Hydroxylgruppe oxyethylierten Pentaerythrits, Tris(2-hydroxyethyl)isocyanurattri(meth)acrylat, Trivinyltrimellitat, Tri(meth)allylamin, Di(meth)allylalkylamine, beispielsweise Di(meth)allylmethylamin, Tri(meth)allylphosphat Tetra(meth)allylethylendiamin, Poly(meth)allylester, Tetra(meth)allyloxiethan oder Tetra(meth)allylammoniumhalide.

Als Verbindung der Vernetzerklasse II sind Verbindungen bevorzugt, die mindestens zwei funktionelle Gruppen aufweisen, die in einer Kondensationsreaktion (=Kondensationsvernetzer), in einer Additionsreaktion oder in einer Ringöffnungsreaktion mit den funktionellen Gruppen der Monomere (α1) oder (α2), bevorzugt mit Säuregruppen, der Monomeren (α1), reagieren können. Bei diesen funktionellen Gruppen der Verbindungen der Vernetzerklasse II handelt es sich vorzugsweise um Alkohol-, Amin-, Aldehyd-, Glycidyl-, Isocyanat-, Carbonat- oder Epichlorfunktionen.

Als Verbindung der Vernetzerklasse II seien als Beispiele genannt Polyole, beispielsweise Ethylenglykol, Polyethylenglykole wie Diethylenglykol, Triethylenglykol und Tetraethylenglykol, Propylenglykol, Polypropylenglykole wie Dipropylenglykol, Tripropylenglykol oder Tetrapropylenglykol, 1,3-Butandiol, 1,4-Butandiol, 1,5-Pentandiol, 2,4-Pentandiol, 1,6-Hexandiol, 2,5-Hexandiol, Glycerin, Polyglycerin, Trimethylolpropan, Polyoxypropylen, Oxyethylen-Oxypropylen-Blockcopolymere, Sorbitanfettsäureester, Polyoxyethylensorbitanfettsäureester, Pentaerythrit, Polyvinylalkohol und Sorbitol, Aminoalkohole, beispielsweise Ethanolamin, Diethanolamin, Triethanolamin oder Propanolamin, Polyaminverbindungen, beispielsweise Ethylendiamin, Diethylentriamin, Triethylentetraamin, Tetraethylenpentaamin oder Pentaethylenhexaamin, Polyglycidylether-Verbindungen wie Ethylenglykoldiglycidylether, Polyethylenglykoldiglycidylether, Glycerindiglycidylether, Glycerinpolyglycidylether, Pentareritritpolyglycidylether, Propylenglykoldiglycidylether, Polypropylenglykoldiglycidylether, Neopentylglykoldiglycidylether, Hexandiolglycidylether, Trimethylolpropanpolyglycidylether, Sorbitolpolyglycidylether, Phthalsäurediglycidylester, Adipinsäurediglycidylether, 1,4-Phenylen-bis(2-oxazolin), Glycidol, Polyisocyanate, vorzugsweise Diisocyanate wie 2,4-Toluoldiisocyanat und Hexamethylendiisocyanat, Polyaziridin-Verbindungen wie 2,2-Bishydroxymethylbutanol-tris[3-(1-aziridinyl)propionat], 1,6-Hexamethylendiethylenharnstoff und Diphenylmethan-bis-4,4'-N,N-diethylenharnstoff, Halogenepoxide beispielsweise Epichlor- und Epibromhydrin und $\alpha$-Methylepichlorhydrin, Alkylencarbonate wie 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on, polyquartäre Amine wie Kondensationsprodukte von Dimethylaminen und Epichlorhydrin. Als Verbindungen der Vernetzerklasse II sind des weiteren Polyoxazoline wie 1,2-Ethylenbisoxazolin, Vernetzer mit Silangruppen wie $\gamma$-Glycidoxypropyltrimethoxysilan und $\gamma$-Aminopropyltrimethoxysilan, Oxazolidinone wie 2-Oxazolidinon, Bis- und Poly-2-oxazolidinone und Diglykolsilikate bevorzugt.

Als Verbindungen der Klasse III sind hydroxyl- oder aminogruppenhaltige Ester der (Meth)acrylsäure, wie beispielsweise 2-Hydroxyethyl(meth)acrylat, sowie hydroxyl- oder aminogruppenhaltige (Meth)acrylamide, oder Mono(meth)allylverbindungen von Diolen bevorzugt.

Die polyvalenten Metallkationen der Vernetzerklasse IV leiten sich vorzugsweise von ein- oder mehrwertigen Kationen ab, die einwertigen insbesondere von Alkalimetallen, wie Kalium, Natrium, Lithium, wobei Lithium bevorzugt wird. Bevorzugte zweiwertige Kationen leiten sich von Zink, Beryllium, Erdalkalimetallen, wie Magnesium, Calcium, Strontium ab, wobei Magnesium bevorzugt wird. Weiter erfindungsgemäß einsetzbare höherwertige Kationen sind Kationen von Aluminium, Eisen, Chrom, Mangan, Titan, Zirkonium und andere Übergangsmetalle sowie Doppelsalze solcher Kationen oder Mischungen der genannten Salze. Bevorzugt werden Aluminiumsalze und Alaune und deren unterschiedliche Hydrate wie z. B. $AlCl_3 \times 6 H_2O$, $NaAl(SO_4)_2 \times 12 H_2O$, $KAl(SO_4)_2 \times 12 H_2O$ oder $Al_2(SO_4)_3 \times 14-18 H_2O$ eingesetzt.

Besonders bevorzugt werden $Al_2(SO_4)_3$ und seine Hydrate als Vernetzer der Vernetzungsklasse IV verwendet. Bevorzugte absorbierende Polymergebilde (Pu1) oder (Pu2) sind Polymergebilde, die durch Vernetzer der folgenden Vernetzerklassen bzw. durch Vernetzer der folgenden Kombinationen von Vernetzerklassen vernetzt sind: I, II, III, IV, I II, I III, I IV, I II III, I II IV, I III IV, II III IV, II IV oder III IV. Die vorstehenden Kombinationen von Vernetzerklassen stellen jeweils eine bevorzugte Ausfiihrungsform von Vernetzern eines Polymer dar.

Weitere bevorzugte Ausführungsformen der absorbierenden Polymergebilde (Pu1) oder (Pu2) sind Polymergebilde, die durch einen beliebigen der vorstehend genannten Vernetzer der Vernetzerklassen I vernetzt sind. Unter diesen sind wasserlösliche Vernetzer bevorzugt. In diesem Zusammenhang sind N,N'-Methylenbisacrylamid, Polyethylenglykoldi(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat besonders bevorzugt.

Als wasserlösliche Polymere ($\alpha$4) können in den erfindungsgemäßen absorbierenden Polymergebilden (Pu1) oder (Pu2) wasserlösliche Polymerisate, wie teil- oder vollverseifter Polyvinylalkohol, Polyvinylpyrrolidon, Stärke oder Stärkederivate, Polyglykole oder Polyacrylsäure enthalten, vorzugsweise einpolymerisiert sein. Das Molekulargewicht dieser Polymere ist unkritisch, solange sie wasserlöslich sind. Bevorzugte wasserlösliche Polymere sind Stärke oder Stärkederivate oder Polyvinylalkohol. Die wasserlöslichen Polymere, vorzugsweise synthetische wie Polyvinylalkohol, können auch als Pfropfgrundlage für die zu polymerisierenden Monomeren dienen.

Als Hilfsstoffe ($\alpha$5) können in den im erfindungsgemäßen Verfahren eingesetzten absorbierenden Polymergebilden (Pu1) oder (Pu2) vorzugsweise Stellmittel, oberflächenaktive Mittel, Geruchsbinder, Füllmittel oder Antioxidatien enthalten sein.

Es ist erfindungsgemäß besonders bevorzugt, dass das absorbierende Polymergebilde (Pu1) oder (Pu2) ein vernetztes Polyacrylat in partikulärer Form ist, welches durch Polymerisation einer Acrylsäure und gegebenenfalls eines der vorstehend genannten Vernetzer in wässrige Lösung, beinhaltend die Acrylsäure in einer Menge in einem Bereich von 5 bis 80 Gew.-%, vorzugsweise 10 bis 70 Gew.-% und besonders bevorzugt 20 bis 50 Gew.-%, bezogen auf das Gewicht der wässrigen Lösung, und anschließendes Zerkleinern des erhaltenen Polymergels, Trocknen des zerkleinerten Gels und gegebenenfalls weiteres Zermahlen des getrockneten Polymergels erhalten wurde. Die auf diese Weise erhaltenen absorbierenden Polymergebilde sind vorzugsweise durch einen Wassergehalt von 0,5 bis 25 Gew.-%, vorzugsweise von 1 bis 10 Gew.-% gekennzeichnet

In den erfindungsgemäßen Verfahren basieren die absorbierenden Polymergebilde (Pu1) oder (Pu2) zu mindestens 50 Gew.-%, bevorzugt zu mindestens 75 Gew.-% und darüber hinaus bevorzugt zu mindestens 90 Gew.-% auf Acrylsäure, die zu mindestens 20 Mol-%, besonders bevorzugt zu mindestens 50 Mol-% neutralisiert ist.

Es ist weiterhin bevorzugt, dass das absorbierende Polymergebilde (Pu1) oder (Pu2) nicht auf Polyacrylnitril-Emulsionen basiert. Dabei ist es bevorzugt, dass die absorbierenden Polymergebilde (Pu1) oder (Pu2) weniger als 37 Mol-%, besonders bevorzugt zu weniger als 20 Mol-%, darüber hinaus bevorzugt zu weniger als 10 Mol-% und darüber hinaus noch mehr bevorzugt zu weniger als 5 Mol-% auf Acrylamid- und/oder Acrylnitril-Monomeren basieren. Es ist in diesem Zusammenhang weiterhin bevorzugt, dass das absorbierende Polymergebilde (Pu1) oder (Pu2) einen Anteil an löslichen, auf Acrylnitril- und/oder Acrylamid-Monomeren basierenden Monomeren oder Polymeren von weniger als 1.000 ppm, besonders bevorzugt weniger als 500 ppm, darüber hinaus bevorzugt weniger als 100 ppm und darüber hinaus noch mehr bevorzugt von weniger als 10 ppm aufweisen.

Aus den vorgenannten Monomeren und Vernetzern lässt sich das absorbierende Polymergebilde (Pu1) oder (Pu2) durch verschiedene Polymerisationsweisen herstellen. Beispielsweise sind in diesem Zusammenhang Massepolymerisation, die vorzugsweise in Knetreaktoren wie Extrudern erfolgt, Lösungspolymerisation, Spraypolymerisation, inverse Emulsionspolymerisation und inverse Suspensionspolymerisation zu nennen. Bevorzugt wird die Lösungspolymerisation in Wasser als Lösungsmittel durchgeführt. Die Lösungspolymerisation kann kontinuierlich oder diskontinuierlich erfolgen. Aus dem Stand der Technik ist ein breites Spektrum von Variationsmöglichkeiten hinsichtlich Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE 27 06 135, US 4,076,663, DE 35 03 458, DE 40 20 780, DE 42 44 548, DE 43 23 001, DE 43 33 056, DE 44 18 818.

Eine andere Möglichkeit zur Herstellung der absorbierenden Polymergebilde (Pu1) oder (Pu2) besteht darin, zunächst unvernetzte, insbesondere lineare Polymere, vorzugsweise auf radikalischem Wege aus den vorgenannten monoethylenisch ungesättigten Monomeren ($\alpha$1) bzw. ($\alpha$2) herzustellen und diese dann mit vernetzend wirkenden Reagenzien ($\alpha$3), vorzugsweise denen der Klassen II und IV, umzusetzen. Diese Variante wird vorzugsweise dann eingesetzt, wenn die Polymergebilde zunächst in formgebenden Verfahren, beispielsweise zu Fasern, Folien oder anderen Flächengebilden, wie Geweben, Gewirken, Gespinsten oder Vliesen verarbeitet und in dieser Form vernetzt werden sollen.

Die Polymerisation wird wie allgemein üblich durch einen Initiator ausgelöst. Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Auch eine Initiierung der Polymerisation durch Einwirkung von Elektronenstrahlen auf die polymerisierbare, wässrige Mischung ist möglich. Die Polymerisation kann allerdings auch in Abwesenheit von Initiatoren der obengenannten Art durch Einwirkung energiereicher Strahlung in Gegenwart von Photoinitiatoren ausgelöst werden. Polymerisationsinitiatoren können in einer Lösung erfindungsgemäßer Monomere gelöst oder dispergiert enthalten sein. Als Initiatoren kommen sämtliche dem Fachmann bekannte in Radikale zerfallende Verbindungen in Betracht. Hierunter fallen insbesondere Peroxide, Hydroperoxide, Wasserstoffperoxid, Persulfate, Azoverbindungen sowie die sogenannten Redoxkatalysatoren. Bevorzugt ist der Einsatz wasserlöslicher Katalysatoren. In manchen Fällen ist es vorteilhaft, Mischungen verschiedener Polymerisationsinitiatoren zu verwenden. Unter diesen Mischungen sind die aus Wasserstoffperoxid und Natrium- oder Kaliumperoxodisulfat bevorzugt, die in jedem denkbaren Mengenverhältnis eingesetzt werden können. Geeignete organische Peroxide sind vorzugsweise Acetylacetonperoxid, Methylethylketonperoxid, t-Butylhydroperoxid, Cumolhydroperoxid, t-Amylperpivalat, t-Butylperpivalat, t-Butylperneohexonat, t-Butylisobutyrat, t-Butylper-2-ethylhexenoat, t-Butylperisononanoat, t-Butylpermaleat, t-Butylperbenzoat, t-Butyl-3,5,5-tri-methylhexanoat und Amylperneodekanoat. Weiterhin sind als Polymerisationsinitiatoren bevorzugt: Azo-Verbindungen, wie 2,2 '-Azobis-(2-amidinopropan)dihydrochlorid, Azo-bis-amidinopropan-dihydrochlorid, 2,2'-Azobis-(N,N-dimethylen)isobutyramidin-dihydrochlorid, 2-(Carbamoylazo)isobutyronitril und 4,4-Azobis-(4-cyanovaleriansäure). Die genannten Verbindungen werden in üblichen Mengen eingesetzt, vorzugsweise in einem Bereich von 0,01 bis 5, bevorzugt von 0,1 bis 2 Mol-%, jeweils bezogen auf die Menge der zu polymerisierenden Monomere.

Die Redoxkatalysatoren enthalten als oxidische Komponente mindestens eine der oben angegebenen Perverbindungen und als reduzierende Komponente vorzugsweise Ascorbinsäure, Glukose, Sorbose, Mannose, Ammonium- oder Alkalimetall-hydrogensulfit, -sulfat, -thiosulfat, -hyposulfit oder -sulfid, Metallsalze, wie Eisen-II-ionen oder Silberionen oder Natriumhydroxymethylsulfoxylat. Vorzugsweise wird als reduzierende Komponente des Redoxkatalysators Ascorbinsäure oder Natriumpyrosulfit verwendet. Bezogen auf die bei der Polymerisation eingesetzte Menge an Monomeren wird $1 \times 10^{-5}$ bis 1 Mol-% der reduzierenden Komponente des Redoxkatalysators und $1 \times 10^{-5}$ bis 5 Mol-% der oxidierenden Komponente des Redoxkatalysators eingesetzt. Anstelle der oxidierenden Komponente des Redoxkatalysators, oder in Ergänzung zu diesem, können ein oder mehrere, vorzugsweise wasserlösliche, Azoverbindungen verwendet werden.

Wenn man die Polymerisation durch Einwirkung energiereicher Strahlung auslöst, verwendet man üblicherweise als Initiator sogenannte Photoinitiatoren. Hierbei kann es sich beispielsweise um sogenannte $\alpha$-Spalter, H-abstrahierende Systeme oder auch um Azide handeln. Beispiele für solche Initiatoren sind Benzophenon-Derivate wie Michlers-Keton, Phenanthren-Derivate, Fluoren-Derivate, Anthrachinon-Derivate, Thioxanton-Derivate, Cumarin-Derivate, Benzoinether und deren Derivate, Azoverbindungen wie die oben genannten Radikalbildner, substituierte Hexaarylbisimidazole oder Acylphosphinoxide. Beispiele für Azide sind: 2-(N,N-Dimethylamino)-ethyl-4-azidocinnamat, 2-(N,N-Dimethylamino)-ethyl-4-azidonaphthylketon, 2-(N,N-Dimethylamino)-ethyl-4-azidobenzoat, 5-Azido-1-naphthyl-2'-(N,N-dimethylamino)ethylsulfon, N-(4-Sulfonylazidophenyl)maleinimid, N-Acetyl-4-sulfonylazidoanilin, 4-Sulfonylazidoanilin, 4-Azidoanilin, 4-Azidophenacylbromid, p-Azidobenzoesäure, 2,6-Bis(p-azidobenzyliden)cyclohexanon und 2,6-Bis-(p-azidobenzyliden)-4-methylcyclohexanon. Die Photoinitiatoren werden, falls sie eingesetzt werden, üblicherweise in Mengen von 0,01 bis 5 Gew.-%, bezogen auf die zu polymerisierenden Monomeren angewendet.

Bevorzugt wird erfindungsgemäß ein Redoxsystem bestehend aus Wasserstoffperoxid, Natriumperoxodisulfat und Ascorbinsäure eingesetzt. Allgemein sind erfindungsgemäß Azoverbindungen als Initiatoren bevorzugt, wobei Azobis-amidinopropan-dihydrochlorid besonders bevorzugt ist. In der Regel wird die Polymerisation mit den Initiatoren in einem Temperaturbereich von 30 bis 90°C initiiert.

Die Trocknung des Polymerisatgels erfolgt bis zu einem Wassergehalt von 0,5 bis 25 Gew.-%, vorzugsweise von 1 bis 10 Gew.-% bei Temperaturen, die üblicherweise im Bereich von 100 bis 200°C liegen.

In einer bevorzugten Ausführungsform zeigt das im erfindungsgemäßen Verfahren eingesetzte, absorbierende Polymergebilde (Pu1) oder (Pu2) mindestens eine der folgenden Eigenschaften (ERT = *EDANA Recommended Test*):

(A) die maximale Aufnahme von 0,9 Gew.-%er NaCl-Lösung gemäß ERT 440.1-99 liegt in einem Bereich von mindestens 10 bis 1000, bevorzugt von 15 bis 500 und besonders bevorzugt von 20 bis 300 g/g,

(B) der mit 0,9 Gew.-%er wässriger NaCl-Lösung extrahierbare Anteil gemäß ERT 470.1-99 beträgt weniger als 30, bevorzugt weniger als 20 und besonders bevorzugt weniger als 10 Gew.-%, bezogen auf das absorbierende Polymergebilde (Pu1) oder (Pu2),

(C) die Schüttdichte gemäß ERT 460.1-99 liegt im Bereich von 300 bis 1000, bevorzugt 310 bis 800 und besonders bevorzugt 320 bis 700 g/l,

(D) der pH-Wert gemäß ERT 400.1-99 von 1 g des absorbierenden Polymergebildes (Pu1) oder (Pu2) in 1 l Wasser liegt im Bereich von 4 bis 10, bevorzugt von 5 bis 9 und besonders bevorzugt von 5,5 bis 7,5,

(E) der CRC-Wert nach ERT 441.1-99 liegt im Bereich von 10 bis 100, bevorzugt 15 bis 80 und besonders bevorzugt 20 bis 60 g/g.

Die sich aus den vorstehenden Eigenschaften ergebenden Eigenschaftskombinationen von zwei oder mehr dieser Eigenschaften stellen jeweils bevorzugte Ausführungsformen des erfindungsgemässen Verfahrens dar. Weiterhin als erfindungsgemässe Ausführungsformen besonders bevorzugt sind Verfahren, in denen das absorbierende Polymergebilde (Pu1) oder (Pu2) die nachfolgend als Buchstaben oder Buchstabenkombinationen dargestellten Eigenschaften oder Eigenschaftskombinationen zeigt: A, B, C, D, E, AB, AC, AD, AE, ABC, ABD, ABE, ACD, ACE, ADE, ABCD, ABCE, ABDE, ACDE, ABCDE.

Das in Kontakt bringen des absorbiererenden Polymergebildes (Pu1) oder (Pu2) mit der wässrigen Lösung erfolgt im erfindungsgemäßen Verfahren vorzugsweise durch gutes Vermischen der wässrigen Lösung mit dem absorbierenden Polymergebilde (Pu1) oder (Pu2). Die wässrige Lösung ist erfindungsgemäß frei von organischen Lösungsmitteln, insbesondere frei von mehrwertigen Alkoholen und Polyalkylenglykolethern, besonders bevorzugt frei von Diethylenglycolmonomethylether und 1,3-Butandiol. Unter einer wässrigen Lösung wird eine Lösung verstanden, die zu mindestens 50 Gew.-%, besonders bevorzugt zu mindestens 60 Gew.-%, darüber hinaus bevorzugt zu mindestens 70 Gew.-% und darüber hinaus noch mehr bevorzugt zu mindestens 90 Gew.-%, jeweils bezogen auf die Gesamtmenge aller in der wässrigen Lösung vorhandenen, bei Raumtemperatur flüssigen Komponenten, auf Wasser basiert.

Dabei ist der chemische Vernetzer von vornherein in der wässrigen Lösung enthaltend das Kieselsäuresol enthalten. Es ist jedoch auch möglich, dass der chemische Vernetzer und das Kieselsäuresol getrennt, vorzugsweise jedoch zeitgleich mit dem absorbierenden Polymergebilde (Pu1) oder (Pu2) in Kontakt gebracht werden. In diesem Fall werden vorzugsweise zwei getrennte Lösungen, von denen die eine den chemischen Vernetzer und die andere das Kieselsäuresol enthält, vorzugsweise gleichzeitig mit dem absorbierenden Polymergebilde (Pu1) oder (Pu2) vermischt, wobei jedoch eine homogene Verteilung des chemischen Vernetzers und des Kieselsäuresols gewährleistet sein muss.

Geeignete Mischaggregate zum Aufbringen der Komponenten sind z. B. der Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymergebilde mittels rotierender Messer in schneller Frequenz gemischt wird (Schugi-Mischer). Das absorbierende Polymergebilde (Pu1) oder (Pu2) wird in dem erfindungsgemäßen Verfahren vorzugsweise mit höchstens 20 Gew.-%, besonders bevorzugt mit höchstens 15 Gew.-%, darüber hinaus bevorzugt mit höchstens 10 Gew.-%, darüber hinaus noch mehr bevorzugt mit höchstens 5 Gew.-% Wasser und am allermeisten bevorzugt mit weniger als 3 Gew.-%, jeweils bezogen auf das Gewicht des absorbierenden Polymergebilde (Pu1) oder (Pu2), in Kontakt gebracht.

Bei einem Einsatz von absorbierenden Polymergebilden (Pu1) oder (Pu2) in der Form von vorzugsweise kugelförmigen Teilchen ist es erfindungsgemäß weiterhin bevorzugt, dass das in Kontakt bringen derart erfolgt, dass lediglich der Aussenbereich, nicht jedoch der innere Bereich der teilchenförmigen absorbierenden Polymergebilde mit dem Kieselsäuresol in Kontakt gebracht werden. In diesem Zusammenhang wird als Aussenbereich der Polymergebilde vorzugsweise derjenige Bereich verstanden, der dadurch gekennzeichnet ist, dass der Abstand eines jeden in diesem Bereich liegenden Raumpunktes vom Mittelpunkt des Teilchens mindestens 50%, besonders bevorzugt mindestens 75%, darüber hinaus bevorzugt mindestens 90% und darüber hinaus noch mehr bevorzugt mindestens 95% des Radius der teilchenförmigen absorbierenden Polymergebilde beträgt. Die auf diese Weise erzielte inhomogene Immobilisierung des Kieselsäuresols auf den Polymergebilden wird erfindungsgemäß dadurch erreicht, dass trockene Polymergebilde mit der wässrigen Lösung in Kontakt gebracht werden und zudem nur so geringe Mengen an Wasser eingesetzt werden, dass es nur im Aussenbereich der absorbierenden Polymergebilde zu einer Absorption der wässrigen Flüssigkeit kommt.

Es ist im erfindungsgemäßen Verfahren weiterhin bevorzugt, dass mindestens 30 Gew.-%, besonders bevorzugt mindestens 60 Gew.-% und darüber hinaus bevorzugt mindestens 90 Gew.-% der kolloiddispersen anorganischen Verbindung eine Partikelgröße im Bereich von 1 bis 100, vorzugsweise von 5 bis 80 und darüber hinaus bevorzugt von 6 bis 50 nm aufweist.

Die anorganische Verbindung wird gemäß dem erfindungsgemäßen Verfahren vorzugsweise in einer Menge von 0,001 bis 10 Gew.-%, besonders bevorzugt von 0,01 bis 5 Gew.-% und darüber hinaus bevorzugt von 0,05 bis 1,5 Gew.-%, bezogen auf das absorbierende Polymergebilde (Pu1) oder (Pu2), mit dem absorbierenden Polymergebilde (Pu1) oder (Pu2) in Kontakt gebracht.

Als anorganische Verbindung wird Kieselsäuresol eingesetzt, und es können alle wasserunlöslichen, anorganischen Verbindungen eingesetzt werden, aus denen stabile, kolloiddisperse, vorzugsweise einphasige, wässrige Lösungen erhalten werden können, die bei 20°C und Normaldruck über einen Zeitraum von mindestens 6h, bevorzugt mindestens 24h und besonders bevorzugt mindestens 72h bis hin zu 6 Monaten keine Phasentrennung, wie etwa das Absetzen eines festen, anorganischen Niederschlags, zeigen.

Unter einer kolloiddispersen Lösung wird vorzugsweise eine Lösung verstanden, die Partikel mit einem Partikeldurchmesser in einem Bereich von 100-1000 Å ($10^{-4}$ bis $10^{-5}$ cm) enthält. Diese Lösungen besitzen die Eigenschaft, einen durch die Lösung geschickten Lichtstrahl in alle Richtungen zu streuen, so dass der Gang des Lichtstrahls durch die kolloiddisperse Lösung verfolgt werden kann (Tyndall Effekt, siehe hierzu Hollemann·Wiberg, *Lehrbuch der anorganischen Chemie,* 91.-100. Auflage, de Gruyter-Verlag, Seite 765).

Als kolloiddisperse anorganische Verbindung werden im erfindungsgemäßen Verfahren Polykieselsäure beinhaltende Partikel eingesetzt in Form von Kieselsäuresol. Eine kolloiddisperse Lösung enthaltend solche Partikel (Kieselsäuresol) kann beispielsweise durch vorsichtiges Ansäuern von infolge Hydrolyse alkalische reagierenden Natriumsilicatlösungen erhalten werden oder aber durch Lösen molekularer Kieselsäure in Wasser und eventueller anschließender Stabilisierung der entstehenden kolloiddispersen Lösung. Die genaue Herstellung derartiger Kieselsäuresole ist dem Fachmann bekannt und ist beispielsweise in Jander·Blasius, *"Lehrbuch der analytischen und präparativen anorganischen Chemie"* S. Hirzel Verlag, Stuttgart, beschrieben.

Neben der kolloiddispersen Kieselsäure sind des weiteren Eisen(III)oxid-Hydrat-Sole, Zinn(IV)oxid-Hydrat-Sole oder auf Silberhalogeniden, insbesondere Silberchlorid, basierende Sole als kolloiddisperse anorganische Verbindung besonders bevorzugt einsetzbar.

Unter chemischen Vernetzern, die im erfindungsgemäßen Verfahren in der wässrigen Lösung enthalten sind, werden vorzugsweise Verbindungen verstanden, die mindestens zwei funktionelle Gruppen aufweisen, die mit funktionellen Gruppen eines Polymers in einer Kondensationsreaktion (=Kondensationsvernetzer), in einer Additionsreaktion oder in einer Ringöffnungsreaktion reagieren können oder aber polyvalente Metallkationen, die mittels elektrostatischer Wechselwirkung zwischen dem polyvalenten Metallkation und den funktionellen Gruppen eines Polymers eine Vernetzung des Polymers ermöglichen. Als chemischer Vernetzer zur Nachvernetzung des Aussenbereiches des absorbierenden Polymergebildes (Pu1) oder (Pu2) - auch "Nachvernetzer" genannt - sind im erfindungsgemäßen Verfahren diejenigen bevorzugt, die im Zusammenhang mit den Vernetzern (α3) als Vernetzer der Vernetzerklassen II und IV genannt wurden.

Unter diesen Verbindungen sind als Nachvernetzer erfindungsgemäß eingesetzt Kondensationsvernetzer ausgewählt aus Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerytrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on.

Besonders bevorzugt wird Ethylencarbonat als Nachvernetzer eingesetzt.

Der Nachvernetzer wird im erfindungsgemäßen Verfahren vorzugsweise in einer Menge im Bereich von 0,01 bis 30, besonders bevorzugt 0,1 bis 20 und darüber hinaus bevorzugt von 0,3 bis 5 Gew.-%, bezogen auf das absorbierende Polymergebilde (Pu1) oder (Pu2) eingesetzt.

Nachdem der chemische Vernetzer und die wässrige Lösung enthaltend Kieselsäuresol mit dem absorbierenden Polymergebilde (Pu1) oder (Pu2) in Kontakt gebracht wurden, erfolgt im erfindungsgemäßen Verfahren die Nachvernetzungsreaktion durch Erhitzen des absorbierenden Polymergebildes auf Temperaturen im Bereich von 40 bis 300°C, bevorzugt von 80 bis 250°C und besonders bevorzugt von 150 bis 220°C. Die optimale Zeitdauer der Nacherhitzung kann für die einzelnen Vernetzertypen und kolloiddispersen anorganischen Verbindungen leicht ermittelt werden. Sie wird dadurch begrenzt, wenn das gewünschte Eigenschaftsprofil des Superabsorbers infolge Hitzeschädigung wieder zerstört wird. Die thermische Behandlung kann in üblichen Trocknern oder Öfen durchgeführt werden, beispielhaft seien Drehrohröfen, Wirbelbetttrockner, Tellertrockner, Paddeltrockner oder Infrarottrockner genannt.

Es ist erfindungsgemäß bevorzugt, dass infolge der thermischen Behandlung der Aussenbereich des absorbierenden Polymergebildes stärker vernetzt ist als der Innenbereich und dass durch die thermische Behandlung die anorganische Verbindung im Aussenbereich mindestens teilweise immobilisiert wird. Weiterhin ist in diesem Zusammenhang bevorzugt, dass der Radius des Aussenbereiches kleiner ist als der dreifache Wert des Radius des Innenbereiches.

In einer anderen Ausführungsform des erfindungsgemäßen Verfahrens wird der Aussenbereich der absorbierenden Polymergebilde vor oder nach, vorzugsweise nach, dem in Kontakt bringen mit der wässrigen Lösung beinhaltend den chemischen Vernetzer und das Kieselsäuresol mit einer Verbindung enthaltend $Al^{3+}$-Ionen in Kontakt gebracht. Dabei ist es bevorzugt, dass die Verbindung enthaltend $Al^{3+}$-Ionen in einer Menge in einem Bereich von 0,01 bis 30 Gew.-%, besonders bevorzugt in einer Menge in einem Bereich von 0,1 bis 20 Gew.-% und darüber hinaus bevorzugt in einer Menge in einem Bereich von 0,3 bis 5 Gew.-%, jeweils bezogen auf das Gewicht der absorbierenden Polymergebilde, mit den Polymergebilden in Kontakt gebracht wird.

Das in Kontakt bringen des Aussenbereiches der absorbierenden Polymergebilde mit der $Al^{3+}$-Ionen enthaltenden Verbindung erfolgt vorzugsweise dadurch, dass das absorbierende Polymergebilde (Pa) mit der Verbindung unter trockenen Bedingungen vermischt wird oder aber dadurch, dass die absorbierenden Polymergebilde (Pa) mit einem Fluid umfassend ein Lösemittel, vorzugsweise Wasser, mit Wasser mischbare organische Lösemittel wie etwa Methanol oder Ethanol oder Mischungen aus mindestens zwei davon, sowie die $Al^{3+}$-Ionen enthaltende Verbindung in Kontakt gebracht werden, wobei das in Kontakt bringen vorzugsweise durch Besprühen der Polymerteilchen mit dem Fluid und Vermischen erfolgt. In diesem Zusammenhang ist es weiterhin bevorzugt, dass das in Kontakt bringen der absorbierenden Polymergebilde (Pa) mit dem Fluid enthaltend die $Al^{3+}$-Ionen enthaltende Verbindung in einem zweistufigen Verfahren erfolgt. Dabei umfasst das zweistufige Verfahren einen ersten Mischvorgang, bei dem eine Vielzahl von absorbierenden Polymergebilden mit dem Fluid vermischt wird, und einem zweiten Mischvorgang, bei dem das Fluid im Inneren der Polymerteilchen homogenisiert wird, wobei die Polymerteilchen in dem ersten Mischvorgang mit einer Geschwindigkeit gemischt werden, dass die Bewegungsenergie der einzelnen Polymerteilchen im Mittel größer ist als die Haftungsenergie zwischen den einzelnen Polymerteilchen, und die Polymerteilchen in dem zweiten Mischvorgang werden mit einer geringeren Geschwindigkeit als im ersten Mischvorgang durchmischt.

Durch die Behandlung der absorbierenden Polymergebilde (Pa) mit dem Fluid beinhaltend die $Al^{3+}$-Ionen enthaltende Verbindung durch das vorstehend beschriebene, zweistufige Verfahren können absorbierende Polymergebilde mit verbesserten Absorptionseigenschaften erhalten werden.

Vorzugsweise ist dabei die $Al^{3+}$-Ionen enthaltende Verbindung ohne Berücksichtigung von Kristallwasser in einer Menge in einem Bereich von 0,1 bis 50 Gew.-%, besonders bevorzugt in einer Menge in einem Bereich von 1 bis 30 Gew.-%, jeweils bezogen auf das Gesamtgewicht des Fluids, in dem Fluid enthalten. Es ist weiterhin bevorzugt, dass das Fluid in einer Menge in einem Bereich von 0,01 bis 15 Gew.-%, besonders bevorzugt in einer Menge in einem Bereich von 0,05 bis 6 Gew.-%, jeweils bezogen auf das Gewicht der absorbierenden Polymergebilde (Pa), mit den absorbierenden Polymergebilden (Pa) in Kontakt gebracht wird.

Bevorzugt $Al^{3+}$-Ionen enthaltenden Verbindungen sind $AlCl_3 \times 6H_2O$, $NaAl(SO_4)_2 \times 12\ H_2O$, $KAl(SO_4)_2 \times 12\ H_2O$ oder $Al_2(SO_4)_3 \times 14$-$18\ H_2O$.

Offenbart wird auch ein absorbierendes Polymergebilde (Pa) beinhaltend einen Innenbereich sowie einen den Innenbereich umgebenden Aussenbereich, wobei der Aussenbereich stärker vernetzt ist als der Innenbereich, im Aussenbereich, vorzugsweise nur im Aussenbereich und nicht im inneren Bereich, eine anorganische Verbindung mindestens teilweise immobilisiert ist und wobei das absorbierende Polymergebilde (Pa) mindestens eine der folgenden Eigenschaften aufweist:

($\beta$1) bei einer CRC nach ERT 441.1-99 <26 g/g eine SFC von mindestens $80 \cdot 10^{-7}$, bevorzugt von mindestens $100 \cdot 10^{-7}$ und besonders bevorzugt von mindestens $120 \cdot 10^{-7}\ cm^3 \cdot s \cdot g^{-1}$,

($\beta$2) bei einer CRC nach ERT 441.1-99 im Bereich $\geq$26 bis <27 g/g eine SFC von mindestens $70 \cdot 10^{-7}$, bevorzugt von mindestens $90 \cdot 10^{-7}$ und besonders bevorzugt von mindestens $110 \cdot 10^{-1}\ cm^3 \cdot s \cdot g^{-1}$,

($\beta$3) bei einer CRC nach ERT 441.1-99 im Bereich $\geq$27 bis <28 g/g eine SFC von mindestens $60 \cdot 10^{-7}$, bevorzugt von mindestens $80 \cdot 10^{-7}$ und besonders bevorzugt von mindestens $100 \cdot 10^{-7}\ cm^3 \cdot s \cdot g^{-1}$,

($\beta$4) bei einer CRC nach ERT 441.1-99 im Bereich $\geq$28 bis <29 g/g eine SFC von mindestens $45 \cdot 10^{-7}$, bevorzugt von mindestens $65 \cdot 10^{-7}$ und besonders bevorzugt von mindestens $85 \cdot 10^{-7}\ cm^3 \cdot s \cdot g^{-1}$,

($\beta$5) bei einer CRC nach ERT 441.1-99 im Bereich $\geq$29 bis <30 eine SFC von mindestens $30 \cdot 10^{-7}$, bevorzugt von mindestens $50 \cdot 10^{-7}$ und besonders bevorzugt von mindestens $70 \cdot 10^{-7}\ cm^3 \cdot s \cdot g^{-1}$,

($\beta$6) bei einer CRC nach ERT 441.1-99 im Bereich $\geq$30 bis <31 eine SFC von mindestens $20 \cdot 10^{-7}$, bevorzugt von mindestens $40 \cdot 10^{-7}$ und besonders bevorzugt von mindestens $60 \cdot 10^{-7}\ cm^3 \cdot s \cdot g^{-1}$,

($\beta$7) bei einer CRC nach ERT 441.1-99 im Bereich $\geq$31 eine SFC von mindestens $10 \cdot 10^{-7}$, bevorzugt von mindestens

$20 \cdot 10^{-7}$ und besonders bevorzugt von mindestens $30 \cdot 10^{-7}$ cm$^3 \cdot$s$\cdot$g$^{-1}$.

Die sich aus den vorstehenden Eigenschaften ergebenden Eigenschaftskombinationen von zwei oder mehr dieser Eigenschaften stellen jeweils bevorzugte Ausführungsformen des absorbierenden Polymergebildes (Pa) dar. Weiterhin als Ausführungsformen besonders bevorzugt ist ein absorbierendes Polymergebilde (Pa), welches die nachfolgend als Buchstaben oder Buchstabenkombinationen dargestellten Eigenschaften oder Eigenschaftskombinationen zeigt: β1, β2, β3, β4, β5, β6, β7, wobei β2, β3, β4, β5 und β6 besonders bevorzugt sind.

Es ist erfindungsgemäß weiterhin bevorzugt, dass das absorbierende Polymergebilde (Pa) eine *Absorbency against Pressure* (AAP) nach ERT 442.1-99 bei einem Druck von 50 g/cm$^2$ von mindestens 18 g/g, besonders bevorzugt mindestens 20 g/g und darüber hinaus besonders bevorzugt von mindestens 22 g/g aufweist.

Es ist bei dem absorbierenden Polymergebilde des Weiteren bevorzugt, dass der Radius des Aussenbereiches kleiner ist als der doppelte Wert des Radius des Innenbereiches.

In einer besonders bevorzugten Ausführungsform der absorbierenden Polymergebilde (Pa) wird als Aussenbereich der Polymergebilde vorzugsweise derjenige Bereich verstanden, der dadurch gekennzeichnet ist, dass der Abstand eines jeden in diesem Bereich liegenden Raumpunktes vom Mittelpunkt des Teilchens mindestens 50%, besonders bevorzugt mindestens 75%, darüber hinaus bevorzugt mindestens 90% und darüber hinaus noch mehr bevorzugt mindestens 95% des Radius der teilchenförmigen absorbierenden Polymergebilde beträgt.

Die anorganische Verbindung, die im Aussenbereich des absorbierenden Polymergebildes (Pa) mindestens teilweise immobilisiert ist, kann jede wasserunlösliche, anorganische Verbindung sein, aus der stabile, kolloiddisperse wässrige Lösungen erhalten werden können.

Eine besonders bevorzugte anorganische Verbindung, die im Aussenbereich des absorbierenden Polymergebilde (Pa) mindestens teilweise immobilisiert ist, ist ein Kondensat von Polykieselsäuren.

Es ist weiterhin bevorzugt, dass die vorstehend genannten Merkmale der absorbierenden Polymergebilde (Pa) auch für die durch das eingangs genannte erfindungsgemäße Verfahren erhältlichen absorbierenden Polymergebilde (Pa) gelten.

Gemäß einer erfindungsgemäßen Ausführungsform des erfindungsgemäßen Verfahrens sowie der absorbierenden Polymergebilde (Pa) ist es bevorzugt, dass die nur mit einer Untergrenze angegebenen Werte von Merkmalen eine Obergrenze besitzen, die das 20-fache, vorzugsweise das 10-fache und besonders bevorzugt das 5-fache des am meisten bevorzugten Wertes der Untergrenze besitzen.

**BEISPIELE**

HERSTELLUNG DER UNBEHANDELTEN, ABSORBIERENDEN POLYMERGEBILDE (PU1)

Pulver A

Eine Monomerenlösung bestehend aus 280 g Acrylsäure, die zu 70 Mol% mit Natronlauge neutralisiert wurde, 466,8 g Wasser, 1,4 g Polyethylenglykol-300-diacrylat und 1,68 g Allyloxypolyethylenglykolacrylsäureester wird durch Spülen mit Stickstoff vom gelösten Sauerstoff befreit und auf die Starttemperatur von 4°C abgekühlt. Nach Erreichen der Starttemperatur wurde die Initiatorlösung (0,1 g 2,2'-Azobis-2-amidinpropan-dihydrochlorid in 10 g H$_2$O, 0,3 g Natriumperoxydisulfat in 10 g H$_2$O, 0,07 g 30%ge Wasserstoffperoxidlösung in 1 g H$_2$O und 0,015 g Ascorbinsäure in 2 g H$_2$O) zugesetzt. Nachdem die Endtemperatur von ca. 100°C erreicht war, wurde das entstandene Gel zerkleinert und bei 150°C 90 Minuten lang getrocknet. Das getrocknete Polymerisat wurde grob zerstoßen, gemahlen und auf ein Pulver mit einer Partikelgröße von 150 bis 850 μm gesiebt.

Das Pulver A besitzt eine Retentionskapazität von 28,8 g/g.

Pulver B

Eine Monomerenlösung bestehend aus 280 g Acrylsäure, die zu 70 Mol% mit Natronlauge neutralisiert wurde, 467,6 g Wasser, 0,98 g Polyethylenglykol-300-diacrylat und 1,26 g Allyloxypolyethylenglykolacrylsäureester wird durch Spülen mit Stickstoff vom gelösten Sauerstoff befreit und auf die Starttemperatur von 4°C abgekühlt. Nach Erreichen der Starttemperatur wurde die Initiatorlösung (0,1 g 2,2'-Azobis-2-amidinpropan-dihydrochlorid in 10 g H$_2$O, 0,3 g Natriumperoxydisulfat in 10 g H$_2$O, 0,07 g 30%ge Wasserstoffperoxidlösung in 1 g H$_2$O und 0,015 g Ascorbinsäure in 2 g H$_2$O) zugesetzt. Nachdem die Endtemperatur von ca. 100°C erreicht war, wurde das entstandene Gel zerkleinert und bei 150°C 90 Minuten lang getrocknet. Das getrocknete Polymerisat wurde grob zerstoßen, gemahlen und auf ein Pulver mit einer Partikelgröße von 150 bis 850 μm gesiebt.

Das Pulver B besitzt eine Retentionskapazität von 31,2 g/g.

Pulver C

Eine Monomerenlösung bestehend aus 280 g Acrylsäure, die zu 70 Mol% mit Natronlauge neutralisiert wurde, 468,6 g Wasser, 0,42 g Polyethylenglykol-300-diacrylat und 0,84 g Allyloxypolyethylenglykolacrylsäureester wird durch Spülen mit Stickstoff vom gelösten Sauerstoff befreit und auf die Starttemperatur von 4°C abgekühlt. Nach Erreichen der Starttemperatur wurde die Initiatorlösung (0,1 g 2,2'-Azobis-2-amidinpropan-dihydrochlorid in 10 g $H_2O$, 0,3 g Natriumperoxydisulfat in 10 g $H_2O$, 0,07 g 30%ge Wasserstoffperoxidlösung in 1 g $H_2O$ und 0,015 g Ascorbinsäure in 2 g $H_2O$) zugesetzt. Nachdem die Endtemperatur von ca. 100°C erreicht war, wurde das entstandene Gel zerkleinert und bei 150°C 90 Minuten lang getrocknet. Das getrocknete Polymerisat wurde grob zerstoßen, gemahlen und auf ein Pulver mit einer Partikelgröße von 150 bis 850 $\mu$m gesiebt.

Das Pulver C besitzt eine Retentionskapazität von 37,1 g/g.

Die in den nachfolgenden Beispielen angegebenen Mengen, in denen die einzelnen Komponenten, wie beispielsweise der Nachvernetzer, das Wasser oder das Kieselsäuresol, bei der Behandlung des Aussenbereiches des unbehandelten, absorbierenden Polymergebildes (Pu1) eingesetzt werden, sind als Mengen bezogen auf das Gewicht des unbehandelten, absorbierenden Polymergebildes (Pu1) zu verstehen.

EINFLUSS DER BEHANDLUNG DES AUSSENBEREICHES DER UNBEHANDELTEN ABSORBIERENDEN POLYMERGEBILDE (PU1) AUF DIE RETENTION, DIE PERMEABILITÄT UND DIE ABSORPTION UNTER DRUCK

Beispiel 1:

50 g Pulver A wird mittels eines Krups-Küchenmixers mit einer Lösung aus 0,5 g Ethylencarbonat, 0,42 g Kieselsäuresol (Produkt Levasil® 200 der Bayer AG, Feststoffanteil ca. 30 Gew.-%) und 1,08 g Wasser unter kräftigem Rühren vermischt und anschließend für 30 min. in einem Ofen, der auf 180°C temperiert war, erhitzt.

Beispiel 2:

50 g Pulver A wird mittels eines Krups-Küchenmixers mit einer Lösung aus 0,5 g Ethylencarbonat, 0,84 g Kieselsäuresol (Produkt Levasil® 200 der Bayer AG, Feststoffanteil ca. 30 Gew.-%) und 0,66 g Wasser unter kräftigem Rühren vermischt und anschließend für 30 min. in einem Ofen, der auf 180°C temperiert war, erhitzt.

Beispiel 3:

50 g Pulver B wird mittels eines Krups-Küchenmixers mit einer Lösung aus 0,5 g Ethylencarbonat, 0,42 g Kieselsäuresol (Produkt Levasil® 200 der Bayer AG, Feststoffanteil ca. 30 Gew.-%) und 1,08 g Wasser unter kräftigem Rühren vermischt und anschließend für 30 min. in einem Ofen, der auf 180°C temperiert war, erhitzt.

Beispiel 4:

50 g Pulver B wird mittels eines Krups-Küchenmixers mit einer Lösung aus 0,5 g Ethylencarbonat, 0,84 g Kieselsäuresol (Produkt Levasil® 200 der Bayer AG, Feststoffanteil ca. 30 Gew.-%) und 0,66 g Wasser unter kräftigem Rühren vermischt und anschließend für 30 min. in einem Ofen, der auf 180°C temperiert war, erhitzt.

Beispiel 5:

50 g Pulver C wird mittels eines Krups-Küchenmixers mit einer Lösung aus 0,5 g Ethylencarbonat, 0,42 g Kieselsäuresol (Produkt Levasil® 200 der Bayer AG, Feststoffanteil ca. 30 Gew.-%) und 1,08 g Wasser unter kräftigem Rühren vermischt und anschließend für 30 min. in einem Ofen, der auf 180°C temperiert war, erhitzt.

Vergleichsbeispiel 1:

50 g Pulver A wird mittels eines Krups-Küchenmixers mit einer Lösung aus 0,5 g Ethylencarbonat und 1,5 g Wasser unter kräftigem Rühren vermischt und anschließend für 30 min. in einem Ofen, der auf 180°C temperiert war, erhitzt.

Vergleichsbeispiel 2:

50 g Pulver B wird mittels eines Krups-Küchenmixers mit einer Lösung aus 0,5 g Ethylencarbonat und 1,5 g Wasser unter kräftigem Rühren vermischt und anschließend für 30 min. in einem Ofen, der auf 180°C temperiert war, erhitzt.

Vergleichsbeispiel 3:

Das in Vergleichsbeispiel 2 erhaltene nachvernetzte Polymergebilde wird mit 0,84 g Kieselsäuresol (Produkt Levasil® 200 der Bayer AG, Feststoffanteil ca. 30 Gew.-%) und 0,16 g Wasser unter kräftigem Rühren vermischt. Das Produkt wird anschließend keinem Temperungsschritt unterzogen.

Vergleichsbeispiel 4:

Das in Vergleichsbeispiel 2 erhaltene nachvernetzte Polymergebilde wird mit 0,84 g Kieselsäuresol (Produkt Levasil® 200 der Bayer AG, Feststoffanteil ca. 30 Gew.-%) und 0,16 g Wasser unter kräftigem Rühren vermischt und anschließend für 60 min. in einem Ofen, der auf 100°C temperiert war, erhitzt.

Vergleichsbeispiel 5:

50 g Pulver B wird mittels eines Krups-Küchenmixers mit einer Lösung aus 0,5 g Ethylencarbonat, 0,125 g Aerosil® (pyrogene Kieselsäure der Degussa AG) und 2 g Wasser unter kräftigem Rühren vermischt und anschließend für 30 min. in einem Ofen, der auf 180°C temperiert war, erhitzt. Zur Herstellung der Suspension von Aerosil® in Wasser waren erhöhte Wassermengen erforderlich. Dennoch ließ sich keine gut dosierbare Suspension erhalten, da sich das eingetragene Aerosil® sehr schnell wieder absetzt und eine homogene Dosierung auf das Pulver B nicht möglich ist. Das beschichtete Polymer neigt zur Klumpenbildung und ist inhomogen.

Vergleichsbeispiel 6:

50 g Pulver C wird mittels eines Krups-Küchenmixers mit einer Lösung aus 0,5 g Ethylencarbonat und 1,5 g Wasser unter kräftigem Rühren vermischt und anschließend für 30 min. in einem Ofen, der auf 180°C temperiert war, erhitzt.

Vergleichsbeispiel 7:

50 g Pulver B wird mittels eines Krups-Küchenmixers mit einer Lösung aus 0,25 g Diethylenglykolmonomethylether, 0,25 g Kieselsäuresol (Produkt Levasil® 200 der Bayer AG, Feststoffanteil ca. 30 Gew.-%) und 1,25 g Wasser unter kräftigem Rühren vermischt und anschließend für 3 min. in einem Ofen, der auf 120°C temperiert war, erhitzt. Diese Behandlung entspricht der Behandlung gemäß Beispiel 1 der JP 1994/16822.

Vergleichsbeispiel 8:

50 g Pulver B wird mittels eines Krups-Küchenmixers mit einer Lösung aus 0,25 g 1,3-Butandiol, 0,25 g Kieselsäuresol (Produkt Levasil® 200 der Bayer AG, Feststoffanteil ca. 30 Gew.-%) und 1,25 g Wasser unter kräftigem Rühren vermischt und anschließend für 3 min. in einem Ofen, der auf 120°C temperiert war, erhitzt. Diese Behandlung entspricht der Behandlung gemäß Beispiel 2 der JP 1994/16822.

Die Eigenschaften der in den Beispielen 1 bis 4 sowie in den Vergleichsbeispielen 1 bis 8 erhaltenen absorbierenden Polymergebilde sind in der folgenden Tabelle 1 zusammengestellt.

Die erfindungsgemäß hergestellten absorbierenden Polymergebilde zeigen eine signifikante Steigerung der Permeabilität (SFC) bei gleichbleibender oder sogar erhöhter Retention gegenüber Produkten, deren Aussenbereich in Abwesenheit eines Kieselsäuresols vernetzt wurde (Beispiel 1 bis 4, Vergleichsbeispiele 1 und 2). Eine Nachbehandlung der schon nachvernetzten Polymergebilde mit Kieslsäuresol führt unabhängig von der nachfolgenden thermischen Behandlung nicht zum gewünschten Ergebnis (Vergleichsbeispiel 3, 4).

Der Zusatz von Aerosil 200® bei der Nachvernetzung führt nicht zu vergleichbar guten Superabsorberkenndaten (Vergleichsbeispiel 5). Weiterhin sind erhöhte Mengen Aerosil 200® nicht mehr in einer akzeptablen Wassermenge zu dispergieren und sind somit nicht mehr dispergierbar.

Vergleichsbeispiele 7 und 8 zeigen, dass in den erfindungsgemäßen Beispielen der ungeprüften JP 1994/16822 keine gute Performance der Polymere hinsichtlich ihrer Permeabilität und Retention zu erzielen ist.

Tabelle 1

| | SFC ($10^{-7} \cdot cm^3 \cdot s \cdot g^{-1}$) | AAP bei 50 $g/cm^2$ (g/g) | CRC (g/g) |
|---|---|---|---|
| Beispiel 1 | 140 | 23,5 | 27 |
| Beispiel 2 | 150 | 23,5 | 27,2 |
| Beispiel 3 | 100 | 24 | 29 |
| Beispiel 4 | 110 | 24 | 29 |
| Vergleichsbeispiel 1 (ohne Kieselsäuresol) | 50 | 24,5 | 26,4 |
| Vergleichsbeispiel 2 (ohne Kieselsäuresol) | 30 | 25 | 27,8 |
| Vergleichsbeispiel 3 (nach Nachvernetzen mit Kieselsäuresol) | 25 | 24 | 28,1 |
| Vergleichsbeispiel 4 (nach Nachvernetzen mit Kieselsäuresol und Erhitzen) | 30 | 24 | 28,7 |
| Vergleichsbeispiel 5 (pyrogene Kieselsäure) | 55 | 23 | 29 |
| Vergleichsbeispiel 6 (ohne Kieselsäuresol) | 17 | 25 | 31,6 |
| Vergleichsbeispiel 7 (JP 1994/16822) | 0 | 9 | 31,3 |
| Vergleichsbeispiel 8 (JP 1994/16822) | 0 | 9 | 31,2 |

Vergleichsbeispiel 9:

50 g Pulver B wird mittels eines Krups-Küchenmixers mit einer Lösung aus 0,5 g Ethylencarbonat und 1,5 g Wasser unter kräftigem Rühren vermischt. Anschließend wird ein Pressling aus dem mit der wässrigen Lösung in Kontakt gebrachten absorbierenden Polymergebilde hergestellt und es werden dessen Dichte und der zur Zerstörung des Presslings aufzuwendende Druck bestimmt.

Die Eigenschaften der in den Beispielen 5 und 6 sowie im Vergleichsbeispiel 9 mit der wässrigen Lösung in Kontakt gebrachten, absorbierenden Polymergebilde sind in der folgenden Tabelle 2 zusammengestellt:

Tabelle 2

| | Dichte des Preßlings ($kg/cm^3$) | Aufzuwendender Druck (Pascal) |
|---|---|---|
| Beispiel 6 | 486 | 8.795 |
| Beispiel 7 | 474 | 4.575 |
| Vergleichsbeispiel 9 | 410 | 16.295 |

Die Ergebnisse zeigen, dass die Bildung stabiler Agglomerate durch den Zusatz von Kieselsäuresol signifikant zurückgedrängt wird. Durch diesen Zusatz wird erreicht, dass das unbehandelte, absorbierende Polymergebilde (Pu1) mit erhöhten Flüssigkeitsmengen beaufschlagt werden kann, ohne dass durch Verklumpen die Verarbeitbarkeit beeinträchtigt wird.

**TESTMETHODEN**

PERMEABILITÄT IM GEQUOLLENEN ZUSTAND (SFC-TEST)

Die Bestimmung der Permeabilität im gequollenen Zustand (*Saline Flow Conductivity* = SFC) erfolgt nach einer in WO 95/22356 beschriebenen Methode. In einem Zylinder mit Siebboden werden ca. 0,9 g Superabsorbermaterial eingewogen und sorgfältig auf der Siebfläche verteilt. Das Superabsorbermaterial lässt man in JAYCO synthetischem Urin 1 Stunde lang gegen einen Druck von 20 $g/cm^2$ quellen. Nach Erfassung der Quellhöhe des Superabsorbers lässt man bei konstantem hydrostatischem Druck 0,118 M NaCl-Lösung aus einem nivellierten Vorratsgefäß durch die gequollene Gelschicht laufen. Die gequollene Gelschicht ist während der Messung mit einem speziellen Siebzylinder abgedeckt, der eine gleichmässige Verteilung der 0,118 M NaCl-Lösung oberhalb des Gels und konstante Bedingungen (Messtemperatur 20-25°C) während der Messung bezüglich der Gelbett-Beschaffenheit gewährleistet. Der auf den gequollenen Superabsorber wirkende Druck ist weiterhin 20 g/cm$^2$. Mit Hilfe eines Computers und einer Waage wird die Flüssigkeitsmenge, die die Gelschicht als Funktion der Zeit passiert, in Intervallen von 20 Sekunden innerhalb einer Zeitperiode von 10 Minuten erfasst. Die Fliessrate g/s durch die gequollene Gelschicht wird mittels Regressionsanalyse mit Extrapolation der Steigung und Ermittlung des Mittelpunktes auf den Zeitpunkt t=0 der Fließmenge innerhalb der Minuten 2-10 ermittelt. Der SFC-Wert (K) wurde in cm$^3$·s·g$^{-1}$ angegeben und wie folgt berechnet:

$$K = \frac{F_s(t=0) \cdot L_o}{r \cdot A \cdot \Delta P_1} = \frac{F_s(t=0) \cdot L_o}{139506}$$

wobei

$F_s(t=0)$     die Fließrate in g/s,
$L_o$     die Dicke der Gelschicht in cm,
$r$     die Dichte der NaCl-Lösung (1,003 g/cm$^3$),
$A$     die Fläche der Oberseite der Gelschicht im Messzylinder (28,27 cm$^2$),
$\Delta P$     der hydrostatische Druck, der auf der Gelschicht lastet (4.920 dyne/cm$^2$), und
$K$     der SFC-Wert ist.

BESTIMMUNG DER AGGLOMERATIONSNEIGUNG

Die Neigung von flüssigkeitsbeschichteten Superabsorbern zur Bildung von Agglomeraten wird mit einem *Indiciser* der Firma J. R. Johanson Inc. bestimmt. Dazu wird der Superabsorber mit der zu untersuchenden Nachvernetzerlösung beschichtet und anschließend 50 g des Pulvers der Untersuchung zugeführt. Das Gerät fertigt mit einem definierten Druck von 160.000 Pascal mittels eines Pressstempels in einem Metallhohlzylinder, der einen Innendurchmesser von 5,23 cm aufweist, einen Preßling mit einer Höhe von etwa 2 cm an. Dieser Preßling wird anschließend durch das Hindurchführen eines zweiten Zylinders, der einen Durchmesser von 4,2 cm aufweist, wieder zerstört, wobei die dazu aufgewendete Kraft gemessen wird.

**Patentansprüche**

1. Verfahren zur Herstellung eines absorbierenden Polymergebildes (Pa) durch Behandeln des Aussenbereiches eines unbehandelten absorbierenden Polymergebildes (Pu1), welches zumindest zu 50 Gew.% auf Acrylsäure basiert, die zu mindestens 20 Mol% neutralisiert ist, umfassend die Schritte:

    - in Kontakt bringen des Aussenbereiches des unbehandelten, absorbierenden Polymergebildes (Pu1) mit einer wässrigen Lösung, die frei ist von organischen Lösungsmitteln ist, die zu mindestens 50 Gew.-%, bezogen auf die Gesamtmenge aller in der wässrigen Lösung vorhandenen, bei Raumtemperatur flüssigen Komponenten, auf Wasser basiert, enthaltend mindestens einen chemischen Vernetzer und Kieselsäuresol;
    wobei als chemischer Vernetzer ein Kondensationsvernetzer eingesetzt wird, welcher ausgewählt ist aus der Gruppe bestehend aus Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerytrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on;
    - Erhitzen des absorbierenden Polymergebildes, dessen Aussenbereich mit der wässrigen Lösung in Kontakt gebracht wurde, auf eine Temperatur im Bereich von 40 bis 300°C, so dass der Aussenbereich des absorbierenden Polymergebildes im Vergleich zum Innenbereich stärker vernetzt ist und die anorganische Verbindung im Aussenbereich des absorbierenden Polymergebildes mindestens teilweise immobilisiert wird.

2. Verfahren zur Herstellung eines absorbierenden Polymergebildes (Pa) durch Behandeln des Aussenbereiches eines nicht mit einer anorganischen Verbindung in kolloiddisperser Form behandelten absorbierenden Polymergebildes 45 (Pu2), welches zumindest zu 50 Gew.% auf Acrylsäure basiert, die zu mindestens 20 Mol% neutralisiert ist, umfassend die Schritte:

- in Kontakt bringen des Aussenbereiches des absorbierenden Polymergebildes (Pu2) mit einer wässrigen Lösung, die frei ist von organischen Lösungsmitteln ist, die zu mindestens 50 Gew.-%, bezogen auf die Gesamtmenge aller in der wässrigen Lösung vorhandenen, bei Raumtemperatur flüssigen Komponenten, auf Wasser basiert, enthaltend mindestens einen chemischen Vernetzer und Kieselsäuresol; wobei als chemischer Vernetzer ein Kondensationsvernetzer eingesetzt wird, welcher ausgewählt ist aus der Gruppe bestehend aus Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerytrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on;
- Erhitzen des absorbierenden Polymergebildes, dessen Aussenbereich mit der wässrigen Lösung in Kontakt gebracht wurde, auf eine Temperatur im Bereich von 40 bis 300°C, so dass der Aussenbereich des absorbierenden Polymergebildes im Vergleich zum Innenbereich stärker vernetzt ist und die anorganische Verbindung im Aussenbereich des absorbierenden Polymergebildes mindestens teilweise immobilisiert wird.

3. Verfahren nach Anspruch 1 oder 2, wobei das absorbierende Polymergebilde (Pu1) oder (Pu2) auf:

   ($\alpha$1) 20-99,999 Gew.-% polymerisierten, ethylenisch ungesättigten, säuregruppenhaltigen Monomeren oder deren Salze oder polymerisierten, ethylenisch ungesättigten, einen protonierten oder quartemierten Stickstoff beinhaltenden Monomeren, oder deren Mischungen,
   ($\alpha$2) 0-80 Gew.-% polymerisierten, monoethylenisch ungesättigten, mit ($\alpha$1) copolymerisierbaren Monomeren,
   ($\alpha$3) 0,001-5 Gew.-% eines oder mehrerer Vernetzer,
   ($\alpha$4) 0-30 Gew.-% eines wasserlöslichen Polymeren, sowie (a5) 0-20 Gew.-% eines oder mehrerer Hilfsmittel basiert, wobei die Summe der Gewichtsmengen ($\alpha$1) bis
   ($\alpha$5) 100 Gew.-% beträgt.

4. Verfahren nach einem der vorhergehenden Ansprüche, wobei das absorbierende Polymergebilde (Pu1) oder (Pu2) mindestens eine der folgenden Eigenschaften aufweist:

   (A) die maximale Aufnahme von 0,9 Gew.-%er NaCl-Lösung liegt in einem Bereich von mindestens 10 bis 1000 g/g,
   (B) der mit 0,9 Gew.-%er wässriger NaCl-Lösung extrahierbare Anteil beträgt weniger als 30 Gew.-%, bezogen auf das absorbierende Polymergebilde (Pu1) oder (Pu2),
   (C) die Schüttdichte liegt im Bereich von 300 bis 1000 g/l,
   (D) der pH-Wert von 1 g des absorbierenden Polymergebildes (Pu1) oder (Pu2) in 1 l Wasser liegt im Bereich von 4 bis 10,
   (E) der CRC-Wert liegt im Bereich von 10 bis 100 g/g.

5. Verfahren nach einem der vorhergehenden Ansprüche, wobei das absorbierende Polymergebilde (Pu1) oder (Pu2) mit höchstens 20 Gew.-% wässriger Lösung, bezogen auf das Gewicht des absorbierenden Polymergebildes (Pu1) oder (Pu2), in Kontakt gebracht wird.

6. Verfahren nach einem der vorhergehenden Ansprüche, wobei zwei getrennte wässrige Lösungen, von denen die eine den chemischen Vernetzer und die andere die anorganische Verbindung in kolloiddisperser Form enthält, zeitgleich mit dem absorbierenden Polymergebilde (Pu1) oder (Pu2) in Kontakt gebracht werden.

7. Verfahren nach einem der vorhergehenden Ansprüche, wobei mindestens 30 Gew.-% der anorganischen Verbindung in der wässrigen Lösung, mit welcher der Aussenbereich des absorbierenden Polymergebildes (Pu1) oder (Pu2) in Kontakt gebracht wird, als Partikel mit einer Partikelgröße im Bereich von 1 bis 100 nm vorliegen.

8. Verfahren nach einem der vorhergehenden Ansprüche, wobei die anorganische Verbindung in einer Menge von 0,001 bis 10 Gew.-%, bezogen auf das absorbierende Polymergebilde (Pu1) oder (Pu2), zur Behandlung des Aussenbereiches des absorbierenden Polymergebildes (Pu1) oder (Pu2) eingesetzt wird.

**Claims**

1. Process for producing an absorbent polymer structure (Pa) by treating the outer region of an untreated absorbent polymer structure (Pu1) based to at least 50 wt.% on acrylic acid neutralized to at least 20 mol%, comprising the steps of:

   - bringing the outer region of the untreated absorbent polymer structure (Pu1) into contact with an aqueous solution free from organic solvents, based to at least 50 wt.%, relative to the overall amount of all room temperature liquid components present in the aqueous solution, on water and comprising at least one chemical crosslinker and silica sol;
   - wherein the chemical crosslinker used is a condensation crosslinker selected from the group consisting of diethylene glycol, triethylene glycol, polyethylene glycol, glycerol, polyglycerol, propylene glycol, diethanolamine, triethanolamine, polyoxypropylene, oxyethylene-oxypropylene block copolymers, sorbitan fatty acid esters, polyoxyethylenesorbitan fatty acid esters, trimethylolpropane, pentaerythritol, polyvinyl alcohol, sorbitol, 1,3-dioxolan-2-one (ethylene carbonate), 4-methyl-1,3-dioxolan-2-one (propylene carbonate), 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, 4,6-dimethyl-1,3-dioxan-2-one, 1,3-dioxolan-2-one, poly-1,3-dioxolan-2-one;
   - heating the absorbent polymer structure whose outer region has been brought into contact with the aqueous solution to a temperature in the range from 40 to 300°C, so the degree of crosslinking of the outer region of the absorbent polymer structure is higher than in the inner region and the inorganic compound becomes at least partially immobilized in the outer region of the absorbent polymer structure.

2. Process for producing an absorbent polymer structure (Pa) by treating the outer region of an absorbent polymer structure 45 (Pu2) untreated with an inorganic compound in colloidally disperse form based to at least 50 wt.% on acrylic acid neutralized to at least 20 mol%, comprising the steps of:

   - bringing the outer region of the absorbent polymer structure (Pu2) into contact with an aqueous solution free from organic solvents, based to at least 50 wt.%, relative to the overall amount of all room temperature liquid components present in the aqueous solution, on water and comprising at least one chemical crosslinker and silica sol;
   wherein the chemical crosslinker used is a condensation crosslinker selected from the group consisting of diethylene glycol, triethylene glycol, polyethylene glycol, glycerol, polyglycerol, propylene glycol, diethanolamine, triethanolamine, polyoxypropylene, oxyethylene-oxypropylene block copolymers, sorbitan fatty acid esters, polyoxyethylenesorbitan fatty acid esters, trimethylolpropane, pentaerythritol, polyvinyl alcohol, sorbitol, 1,3-dioxolan-2-one (ethylene carbonate), 4-methyl-1,3-dioxolan-2-one (propylene carbonate), 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, 4,6-dimethyl-1,3-dioxan-2-one, 1,3-dioxolan-2-one, poly-1,3-dioxolan-2-one;
   - heating the absorbent polymer structure whose outer region has been brought into contact with the aqueous solution to a temperature in the range from 40 to 300°C, so the degree of crosslinking of the outer region of the absorbent polymer structure is higher than in the inner region and the inorganic compound becomes at least partially immobilized in the outer region of the absorbent polymer structure.

3. Process according to Claim 1 or 2, wherein the absorbent polymer structure (Pu1) or (Pu2) is based on:

   ($\alpha$1) 20-99.999 wt.% of polymerized ethylenically unsaturated acid-functional monomers or their salts or polymerized ethylenically unsaturated monomers comprising a protonated or quaternized nitrogen, or mixtures thereof,
   ($\alpha$2) 0-80 wt.% of polymerized ethylenically unsaturated monomers copolymerizable with ($\alpha$1),
   ($\alpha$3) 0.001-5 wt.% of one or more crosslinkers,
   ($\alpha$4) 0-30 wt.% of a water-soluble polymer, and also
   ($\alpha$5) 0-20 wt.% of one or more auxiliaries, wherein the sum total of weight quantities ($\alpha$1) to ($\alpha$5) is 100 wt.%.

4. Process according to any preceding claim, wherein the absorbent polymer structure (Pu1) or (Pu2) has at least one of the following properties:

   (A) the maximum absorption of 0.9 wt.% NaCl solution is within a range from at least 10 to 1000 g/g,

(B) the part extractable with 0.9 wt.% aqueous NaCl solution amounts to less than 30 wt.%, relative to the absorbent polymer structure (Pu1) or (Pu2),

(C) the bulk density is within the range from 300 to 1000 g/l,

(D) the pH value for 1 g of the absorbent polymer structure (Pu1) or (Pu2) in 1 l of water is within the range from 4 to 10,

(E) the CRC value is within the range from 10 to 100 g/g.

5. Process according to any preceding claim, wherein the absorbent polymer structure (Pu1) or (Pu2) is brought into contact with at most 20 wt.% of aqueous solution, relative to the weight of the absorbent polymer structure (Pu1) or (Pu2).

6. Process according to any preceding claim, wherein two separate aqueous solutions, of which one contains the chemical crosslinker and the other the inorganic compound in colloidally disperse form, are simultaneously brought into contact with the absorbent polymer structure (Pu1) or (Pu2).

7. Process according to any preceding claim, wherein at least 30 wt.% of the inorganic compound is present as particles having a particle size in the range from 1 to 100 nm in the aqueous solution which is brought into contact with the outer region of the absorbent polymer structure (Pu1) or (Pu2).

8. Process according to any preceding claim, wherein the inorganic compound is used in an amount of 0.001 to 10 wt.%, relative to the absorbent polymer structure (Pu1) or (Pu2), for treating the outer region of the absorbent polymer structure (Pu1) or (Pu2).

**Revendications**

1. Procédé de fabrication d'une structure polymère absorbante (Pa) par traitement de la zone extérieure d'une structure polymère absorbante non traitée (Pu1), qui est au moins à 50 % en poids à base d'acide acrylique, qui est neutralisé à au moins 20 % en moles, comprenant les étapes suivantes :

- la mise en contact de la zone extérieure de la structure polymère absorbante non traitée (Pu1) avec une solution aqueuse, qui est exempte de solvants organiques, qui est au moins à 50 % en poids, par rapport à la quantité totale de tous les composants liquides à température ambiante présents dans la solution aqueuse, à base d'eau, contenant au moins un agent de réticulation chimique et un sol de silice ;

dans lequel procédé on utilise comme agent de réticulation chimique un agent de réticulation par condensation choisi parmi le groupe consistant en le diéthylène glycol, le triéthylène glycol, le polyéthylène glycol, la glycérine, la polyglycérine, le propylène glycol, la diéthanolamine, la triéthanolamine, le polyoxypropylène, les copolymères séquencés d'oxyéthylène-oxypropylène, les esters d'acides gras de sorbitane, les esters d'acides gras de polyoxyéthylène-sorbitane, le triméthylolpropane, la pentaérythrite, l'alcool polyvinylique, le sorbitol, la 1,3-dioxolan-2-one (carbonate d'éthylène), la 4-méthyl-1,3-dioxolan-2-one (carbonate de propylène), la 4,5-diméthyl-1,3-dioxolan-2-one, la 4,4-diméthyl-1,3-dioxolan-2-one, la 4-éthyl-1,3-dioxolan-2-one, la 4-hydroxyméthyl-1,3-dioxolan-2-one, la 1,3-dioxan-2-one, la 4-méthyl-1,3-dioxan-2-one, la 4,6-diméthyl-1,3-dioxan-2-one, la 1,3-dioxolan-2-one, la poly-1,3-dioxolan-2-one ;

- le chauffage de la structure polymère absorbante, dont la zone extérieure a été mise en contact avec la solution aqueuse, à une température dans la plage allant de 40 à 300 °C, de manière à ce que la zone extérieure de la structure polymère absorbante soit plus fortement réticulée en comparaison de la zone intérieure et à ce que le composé inorganique dans la zone extérieure de la structure polymère absorbante soit au moins partiellement immobilisé.

2. Procédé de fabrication d'une structure polymère absorbante (Pa) par traitement de la zone extérieure d'une structure polymère absorbante 45 (Pu2) non traitée avec un composé inorganique sous forme colloïdale dispersée, qui est au moins à 50 % en poids à base d'acide acrylique, qui est neutralisé à au moins 20 % en moles, comprenant les étapes suivantes :

- la mise en contact de la zone extérieure de la structure polymère absorbante (Pu2) avec une solution aqueuse, qui est exempte de solvants organiques, qui est au moins à 50 % en poids, par rapport à la quantité totale de tous les composants liquides à température ambiante présents dans la solution aqueuse, à base d'eau, contenant au moins un agent de réticulation chimique et un sol de silice ;

dans lequel procédé on utilise comme agent de réticulation chimique un agent de réticulation par condensation choisi parmi le groupe consistant en le diéthylène glycol, le triéthylène glycol, le polyéthylène glycol, la glycérine, la polyglycérine, le propylène glycol, la diéthanolamine, la triéthanolamine, le polyoxypropylène, les copolymères séquencés d'oxyéthylène-oxypropylène, les esters d'acides gras de sorbitane, les esters d'acides gras de polyoxyéthylène-sorbitane, le triméthylolpropane, la pentaérythrite, l'alcool polyvinylique, le sorbitol, la 1,3-dioxolan-2-one (carbonate d'éthylène), la 4-méthyl-1,3-dioxolan-2-one (carbonate de propylène), la 4,5-diméthyl-1,3-dioxolan-2-one, la 4,4-diméthyl-1,3-dioxolan-2-one, la 4-éthyl-1,3-dioxolan-2-one, la 4-hydroxyméthyl-1,3-dïoxolan-2-one, la 1,3-dioxan-2-one, la 4-méthyl-1,3-dioxan-2-one, la 4,6-diméthyl-1,3-dioxan-2-one, la 1,3-dioxolan-2-one, la poly-1,3-dioxolan-2-one ;
- le chauffage de la structure polymère absorbante, dont la zone extérieure a été mise en contact avec la solution aqueuse, à une température dans la plage allant de 40 à 300 °C, de manière à ce que la zone extérieure de la structure polymère absorbante soit plus fortement réticulée en comparaison de la zone intérieure et à ce que le composé inorganique dans la zone extérieure de la structure polymère absorbante soit au moins partiellement immobilisé.

3. Procédé selon la revendication 1 ou 2, dans lequel la structure polymère absorbante (Pu1) ou (Pu2) est à base de :

($\alpha$1) 20 à 99,999 % en poids de monomères polymérisés, éthyléniquement insaturés, contenant des groupes acide,

ou leurs sels, ou de monomères polymérisés, éthyléniquement insaturés, contenant un azote protoné ou quaternisé, ou leurs mélanges,

($\alpha$2) 0 à 80 % en poids de monomères polymérisés, monoéthyléniquement insaturés, copolymérisables avec ($\alpha$1),

($\alpha$3) 0,001 à 5 % en poids d'un ou de plusieurs agents de réticulation,

($\alpha$4) 0 à 30 % en poids d'un polymère soluble dans l'eau, et

($\alpha$5) 0 à 20 % en poids d'un ou de plusieurs adjuvants, la somme des quantités en poids de ($\alpha$1) à ($\alpha$5) étant de 100 % en poids.

4. Procédé selon l'une quelconque des revendications précédentes, dans lequel la structure polymère absorbante (Pu1) ou (Pu2) présente au moins une des propriétés suivantes :

(A) l'absorption maximale d'une solution de NaCl à 0,9 % en poids se situe dans une plage allant d'au moins 10 à 1 000 g/g,

(B) la fraction extractible avec une solution aqueuse de NaCl à 0,9 % en poids est inférieure à 30 % en poids, par rapport à la structure polymère absorbante (Pu1) ou (Pu2),

(C) la densité apparente se situe dans la plage allant de 300 à 1 000 g/l,

(D) le pH de 1 g de la structure polymère absorbante (Pu1) ou (Pu2) dans 1 l d'eau se situe dans la plage allant de 4 à 10,

(E) la valeur CRC se situe dans la plage allant de 10 à 100 g/g.

5. Procédé selon l'une quelconque des revendications précédentes, dans lequel la structure polymère absorbante (Pu1) ou (Pu2) est mise en contact avec au plus 20 % en poids de solution aqueuse, par rapport au poids de la structure polymère absorbante (Pu1) ou (Pu2).

6. Procédé selon l'une quelconque des revendications précédentes, dans lequel deux solutions aqueuses séparées, parmi lesquelles une contient l'agent de réticulation chimique et l'autre le composé inorganique sous forme colloïdale dispersée, sont mises en contact simultanément avec la structure polymère absorbante (Pu1) ou (Pu2).

7. Procédé selon l'une quelconque des revendications précédentes, dans lequel au moins 30 % en poids du composé inorganique est présent dans la solution aqueuse, avec laquelle la zone extérieure de la structure polymère absorbante (Pu1) ou (Pu2) est mise en contact, sous la forme de particules ayant une taille de particule dans la plage allant de 1 à 100 nm.

8. Procédé selon l'une quelconque des revendications précédentes, dans lequel le composé inorganique est utilisé en une quantité de 0,001 à 10 % en poids, par rapport à la structure polymère absorbante (Pu1) ou (Pu2), pour le traitement de la zone extérieure de la structure polymère absorbante (Pu1) ou (Pu2).

**IN DER BESCHREIBUNG AUFGEFÜHRTE DOKUMENTE**

**In der Beschreibung aufgeführte Patentdokumente**

- DE 19646484
- EP 0450923 A
- EP 0450922 A
- DE 3523617 A
- US 5140076 A
- US 4734478 A
- DE 3503458
- US 4535098 A
- DE 19805447
- DE 19854575
- US 5147921 A
- EP 1211266 A1
- EP 1211266 A
- WO 0113841 A1
- JP 6016822 A
- DE 19529348
- WO 9934843 A
- DE 19543366
- DE 19543368
- US 4286082 A
- DE 2706135
- US 4076663 A
- DE 4020780
- DE 4244548
- DE 4323001
- DE 4333056
- DE 4418818
- WO 9522356 A